# EUROPEAN PATENT APPLICATION

(11) **EP 4 137 578 A1**
(43) Date of publication of application: **22.02.2023**
(21) Application number: 22175898.0
(22) Date of filing: 04.01.2019
(51) Int. Cl.: C12N 15/863, A61K 35/76, A61K 35/768, A61P 35/00, C07K 14/07, C12N 15/39, C12N 5/10, C12N 7/01

(54) **MODIFIED VACCINIA VECTORS**

(30) Priority: 05.01.2018 US 201862614349 P; 03.07.2018 US 201862693745 P; 21.12.2018 US 201862784370 P
(62) Divisional of application: 19736225.4
(71) Applicant: Ottawa Hospital Research Institute, Ottawa, Ontario K1H 8L6 (CA)
(72) Inventor: BELL, John, Ottawa, K1G 0K8 (CA); LE BOEUF, Fabrice, Gatineau, J9J 1G1 (CA); HUH, Michael S., Ottawa, K1K 4Z9 (CA); TANG, Matthew Y., Orleans, K1C 5Z6 (CA); KELLER, Brian Andrew, Ottawa, K1H 5N9 (CA); PELIN, Adrian, San Francisco, 94107 (US)
(74) Representative: Jones Day

(57) **Abstract**

The disclosure relates to modified vaccinia virus vectors derived from the Copenhagen strain of vaccinia virus, as well as methods of using the same for the treatment of various cancers. The disclosure provides modified Copenhagen-derived vaccinia virus vectors that exhibit various beneficial therapeutic activities, including enhanced oncolytic activity, spread of infection, immune evasion, tumor persistence, capacity for incorporation of exogenous DNA sequences, amenability for large scale manufacturing, and safety.

## Description

### Field

The invention relates to the field of immunotherapy, e.g., for the treatment of cell proliferation disorders, such as cancers. Particularly, the invention relates to genetically modified vaccinia viruses, as well as methods of making and using the same.

### Background

The immune system may be stimulated to identify tumor cells and target them for destruction. Immunotherapy employing oncolytic vaccinia viruses is a rapidly evolving area in cancer research. New approaches are needed to engineer and/or enhance tumor-selectivity for oncolytic viruses in order to maximize efficiency and safety. This selectivity is especially important when potentially toxic therapeutic agents or genes are added to the viruses.

Although the use of vaccinia viruses as clinical oncolytic vectors is a promising paradigm for cancer treatment, due to toxicity, such as pox lesions in patients, and immunosuppressive side effects, most current clinical candidates have shown only modest clinical success. There exists a need for methods to engineer vaccinia viruses that exhibit more robust virus replication, cancer cell killing, and spreading from the point of infection. The present invention addresses this need and provides a solution to selectivity and safety limitations by employing a modified vaccinia virus.

### Summary

The present disclosure describes the use of Copenhagen-derived Vaccinia virus vectors for the treatment of cancer. In particular, the disclosure is based in part on the surprisingly enhanced oncolytic activity, spread of infection, and safety results engendered when a vaccinia virus is genetically modified to contain deletions in some or all, of the following genes: C2L, C1L, N1L, N2L, MIL, M2L, K1L, K2L, K3L, K4L, K5L, K6L, K7R, F1L, F2L, F3L, B14R, B15R, B16R, B17L, B18R, B19R, B20R, K ORF A, K ORF B, B ORF E, B ORF F, B ORF G, B21R, B22R, B23R, B24R, B25R, B26R, B27R, B28R, and B29R. Specifically, a vector derived from the genetically modified Copenhagen-derived vaccinia viruses that exhibit mutations in one or more, or all, of these genes may exhibit an array of beneficial features, such as improved oncolytic ability, replication in tumors, infectivity, immune evasion, tumor persistence, capacity for incorporation of exogenous DNA sequences, and amenability for large scale manufacturing. The present disclosure decribes vaccinia viruses further genetically modified to contain deletions in the B8R gene. In various embodiments disclose below, the invention may further include a deletion of the B8R gene. In various embodiments, the modified vaccinia virus expresses at least one transgene.

In a first aspect, the invention features a nucleic acid that includes a recombinant vaccinia virus genome, wherein the recombinant vaccinia virus genome has a deletion of at least six vaccinia genes, one vaccinia gene from each of the following (a)-(f): (a) F1L; (b)N1L, and B14R; (c) M2L, K1L, and K7R; (d) C2L, N2L, MIL, K2L, K3L, F3L, B16R, and B19R; (e) K4L, K5L, K6L, and F2L; (f) B15R, B17L, B18R, and B20R.

In some embodiments, the deletion includes at least 7, 8, 9, 10, 11, 12, 13, 14, 15, 16, 17, 18, 19, 20, 21 or 22 genes, each independently selected from the group consisting of C2L, C1L, N1L, N2L, MIL, M2L, K1L, K2L, K3L, K4L, K5L, K6L, K7R, F1L, F2L, F3L, B14R, B15R, B16R, B17L, B18R, B19R, B20R. In some embodiments, the deletion includes each of the C2L, C1L, N1L, N2L, MIL, M2L, K1L, K2L, K3L, K4L, K5L, K6L, K7R, F1L, F2L, F3L, B14R, B15R, B16R, B17L, B18R, B19R, B20R genes. In various embodiments, the recombinant vaccinia virus genome may further include a B8R deletion.

In another aspect, the invention features a nucleic acid that includes a recombinant vaccinia virus genome, wherein the recombinant vaccinia virus genome has a deletion of at least 1, 2, 3, 4, or 5 genes selected from the group consisting of B14R, B16R, B17L, B18R, B19R, and B20R. In some embodiments, the deletion includes each of B14R, B16R, B17L, B18R, B19R, and B20R. In various embodiments, the recombinant vaccinia virus genome may further include a B8R deletion.

In another aspect, the invention features a nucleic acid that includes a recombinant vaccinia virus genome, wherein the recombinant vaccinia virus genome has a deletion of at least 1, 2, 3, 4, 5, 6, 7, 8, 9 or 10 genes selected from the group consisting of C2L, C1L, N1L, N2L, MIL, M2L, K1L, K2L, K3L, K4L, K5L, K6L, K7R, F1L, F2L, F3L. In some embodiments, the deletion includes each of C2L, C1L, N1L, N2L, MIL, M2L, K1L, K2L, K3L, K4L, K5L, K6L, K7R, F1L, F2L, F3L. In various embodiments, the recombinant vaccinia virus genome may further include a B8R deletion.

In another aspect, the invention features a nucleic acid that includes a recombinant vaccinia virus genome, wherein the recombinant vaccinia virus genome has a deletion of at least 1 gene that encodes a caspase-9 inhibitor. In some embodiments, the gene that encodes a caspase-9 inhibitor is F1L.

In another aspect, the invention features a nucleic acid that includes a recombinant vaccinia virus genome, wherein the recombinant vaccinia virus genome has a deletion of at least 1 gene that encodes a BCL-2 inhibitor. In some embodiments, the gene that encodes a BCL-2 inhibitor is N1L.

In another aspect, the invention features a nucleic acid that includes a recombinant vaccinia virus genome, wherein the recombinant vaccinia virus genome has a deletion of at least 1 gene that encodes a dUTPase. In some embodiments, the gene that encodes a dUTPase is F2L.

In another aspect, the invention features a nucleic acid that includes a recombinant vaccinia virus genome, wherein the recombinant vaccinia virus genome has a deletion of at least 1 gene that encodes a IFN-alpha/beta-receptor-like secreted glycoprotein. In some embodiments, the gene that encodes a IFN-alpha/beta-receptor-like secreted glycoprotein is B19R.

In another aspect, the invention features a nucleic acid that includes a recombinant vaccinia virus genome, wherein the recombinant vaccinia virus genome has a deletion of at least 1 gene that encodes an IL-1-beta-inhibitor. In some embodiments, the gene that encodes an IL-1-beta-inhibitor is B16R.

In another aspect, the invention features a nucleic acid that includes a recombinant vaccinia virus genome, wherein the recombinant vaccinia virus genome has a deletion of at least 1 gene that encodes a phospholipase-D. In some embodiments, the gene that encodes a phospholipase-D is K4L.

In another aspect, the invention features a nucleic acid that includes a recombinant vaccinia virus genome, wherein the recombinant vaccinia virus genome has a deletion of at least 1 gene that encodes a PKR inhibitor. In some embodiments, the gene that encodes a PKR inhibitor is K3L.

In another aspect, the invention features a nucleic acid that includes a recombinant vaccinia virus genome, wherein the recombinant vaccinia virus genome has a deletion of at least 1 gene that encodes a serine protease inhibitor. In some embodiments, the gene that encodes a serine protease inhibitor is K2L.

In another aspect, the invention features a nucleic acid that includes a recombinant vaccinia virus genome, wherein the recombinant vaccinia virus genome has a deletion of at least 1 gene that encodes a TLR signaling inhibitor. In some embodiments, the gene that encodes a TLR signaling inhibitor is N2L.

In another aspect, the invention features a nucleic acid that includes a recombinant vaccinia virus genome, wherein the recombinant vaccinia virus genome has a deletion of at least 1 or 2 genes that encodes a kelch-like protein. In some embodiments, the genes that encode a kelch-like protein are, independently, selected from the group consisting of F3L and C2L.

In another aspect, the invention features a nucleic acid that includes a recombinant vaccinia virus genome, wherein the recombinant vaccinia virus genome has a deletion of at least 1 or 2 genes that encodes a monoglyceride lipase. In some embodiments, the genes that encode a monoglyceride lipase are, independently, selected from the group consisting of K5L and K6L.

In another aspect, the invention features a nucleic acid that includes a recombinant vaccinia virus genome, wherein the recombinant vaccinia virus genome has a deletion of at least 1, 2 or 3 genes that encodes an NF-κB inhibitor. In some embodiments, the genes that encode an NF-κB inhibitor are independently selected from the group consisting of K7R, K1L, and M2L.

In some embodiments, the recombinant vaccinia virus genome has a deletion of at least 1, 2, or 3 genes that encodes an Ankyrin repeat protein. In some embodiments, the genes that encode an Ankyrin repeat protein are independently selected from the group consisting of B18R, B20R, and M1L.

In some embodiments, the recombinant vaccinia virus genome has a deletion of at least 1, 2 or 3 genes selected from the group consisting of B15R, B17R, and B14RIn various embodiments, the recombinant vaccinia virus genome may further include a B8R deletion.

In some embodiments, the recombinant vaccinia virus genome has a deletion of at least 1, 2, 3, 4, 5, 6, 7, or 8 genes selected from the group of inverted terminal repeat (ITR) genes consisting of B21R, B22R, B23R, B24R, B25R, B26R, B27R, B28R, and B29R. In some embodiments, the deletion includes each of B21R, B22R, B23R, B24R, B25R, B26R, B27R, B28R, and B29R. In various embodiments, the recombinant vaccinia virus genome may further include a B8R deletion. In various embodiments, the recombinant vaccinia virus genome may further include a B8R deletion.

In some embodiments, one or more, or all, of the deletions is a deletion of the entire polynucleotide encoding the corresponding gene. In some embodiments, one or more, or all, of the deletions is a deletion of a portion of the polynucleotide encoding the corresponding gene, such that the deletion is sufficient to render the gene nonfunctional, e.g., upon introduction into a host cell.

In some embodiments, the nucleic acid further includes a transgene that encodes a protein that provides improved oncolytic activity, a protein capable of eliciting an immune response for use as a vaccine, a therapeutic polypeptide or a therapeutic nucleic acid. In some embodiments the transgene encodes a protein that provides improved oncolytic activity. In some embodiments the transgene encodes a protein capable of eliciting an immune response for use as a vaccine. In some embodiments the transgene encodes a protein that provides a therapeutic polypeptide. In some embodiments the transgene encodes a therapeutic polypeptide.

In another aspect, the invention features a recombinant vaccinia virus vector that has a deletion of at least 2, 3, 4, 5, 6, 7, 8, 9, 10, 11, 12, 13, 14, 15, 16, 17, 18, 19, 20, 21 or 22 genes, each independently selected from the group consisting of C2L, C1L, N1L, N2L, MIL, M2L, K1L, K2L, K3L, K4L, K5L, K6L, K7R, F1L, F2L, F3L, B14R, B15R, B16R, B17L, B18R, B19R, B20R, K ORF A, K ORF B, B ORF E, B ORF F, B ORF G, B21R, B22R, B23R, B24R, B25R, B26R, B27R, B28R, and B29R. In some embodiments, the deletion includes each of C2L, C1L, N1L, N2L, MIL, M2L, K1L, K2L, K3L, K4L, K5L, K6L, K7R, F1L, F2L, F3L, B14R, B15R, B16R, B17L, B18R, B19R, B20R, K ORF A, K ORF B, B ORF E, B ORF F, B ORF G, B21R, B22R, B23R, B24R, B25R, B26R, B27R, B28R, and B29R. In various embodiments, the recombinant vaccinia virus genome may further include a B8R deletion.

In another aspect, the invention features a recombinant vaccinia virus vector that includes a recombinant vaccinia virus genome, wherein the recombinant vaccinia virus genome has a deletion of at least 1, 2, 3, 4 or 5 genes selected from the group consisting of B14R, B16R, B17L, B18R, B19R, and B20R. In some embodiments, the deletion includes each of B14R, B16R, B17L, B18R, B19R, and B20R. In various embodiments, the recombinant vaccinia virus genome may further include a B8R deletion.

In another aspect, the invention features a recombinant vaccinia virus vector that includes a recombinant vaccinia virus genome, wherein the recombinant vaccinia virus genome has a deletion of at least 1, 2, 3, 4, 5, 6, 7, 8, 9, 10, 11, 12, 13, 14, 15 or 16 genes selected from the group consisting of C2L, C1L, N1L, N2L, MIL, M2L, K1L, K2L, K3L, K4L, K5L, K6L, K7R, F1L, F2L, F3L In some embodiments, the deletion includes each of C2L, C1L, N1L, N2L, MIL, M2L, K1L, K2L, K3L, K4L, K5L, K6L, K7R, F1L, F2L, F3L. In various embodiments, the recombinant vaccinia virus genome may further include a B8R deletion.

In another aspect, the invention features a recombinant vaccinia virus vector that has a deletion of at least 1 gene that encodes a caspase-9 inhibitor. In some embodiments, the gene that encodes a caspase-9 inhibitor is F1L.

In another aspect, the invention features a recombinant vaccinia virus vector has a deletion of at least 1 gene that encodes a BCL-2 inhibitor. In some embodiments, the gene that encodes a BCL-2 inhibitor is N1L.

In another aspect, the invention features a recombinant vaccinia virus vector has a deletion of at least 1 gene that encodes a dUTPase. In some embodiments, the gene that encodes a dUTPase is F2L.

In another aspect, the invention features a recombinant vaccinia virus vector has a deletion of at least 1 gene that encodes a IFN-alpha/beta-receptor-like secreted glycoprotein. In some embodiments, the gene that encodes a IFN-alpha/beta-receptor-like secreted glycoprotein is B19R.

In another aspect, the invention features a recombinant vaccinia virus vector has a deletion of at least 1 gene that encodes an IL-1-beta-inhibitor. In some embodiments, the gene that encodes an IL-1-beta-inhibitor is B16R.

In another aspect, the invention features a recombinant vaccinia virus vector has a deletion of at least 1 gene that encodes a phospholipase-D. In some embodiments, the gene that encodes a phospholipase-D is K4L.

In another aspect, the invention features a recombinant vaccinia virus vector has a deletion of at least 1 gene that encodes a PKR inhibitor. In some embodiments, the gene that encodes a PKR inhibitor is K3L.

In another aspect, the invention features a recombinant vaccinia virus vector has a deletion of at least 1 gene that encodes a serine protease inhibitor. In some embodiments, the gene that encodes a serine protease inhibitor is K2L.

In another aspect, the invention features a recombinant vaccinia virus vector has a deletion of at least 1 gene that encodes a TLR signaling inhibitor. In some embodiments, the gene that encodes a TLR signaling inhibitor is N2L.

In another aspect, the invention features a recombinant vaccinia virus vector has a deletion of at least 1 gene that encodes a kelch-like protein.

In some embodiments, the recombinant vaccinia virus genome has a deletion of at least 1 or 2 genes that encode a kelch-like protein. In some embodiments, the genes that encode a kelch-like protein are, independently, selected from the group consisting of F3L and C2L.

In another aspect, the invention features a recombinant vaccinia virus vector has a deletion of at least 1 gene that encodes a monoglyceride lipase.

In some embodiments, the recombinant vaccinia virus genome has a deletion of at least 1 or 2 genes that encode a monoglyceride lipase. In some embodiments, the genes that encode a monoglyceride lipase are, independently, selected from the group consisting of K5L and K6L.

In another aspect, the invention features a recombinant vaccinia virus vector has a deletion of at least 1 gene that encodes an NF-κB inhibitor.

In some embodiments, the recombinant vaccinia virus genome has a deletion of at least 1, 2 or 3 genes that encode an NF-κB inhibitor. In some embodiments, the genes that encode an NF-κB inhibitor are, independently, selected from the group consisting of K7R, K1L, and M2L.

In another aspect, the invention features a recombinant vaccinia virus vector has a deletion of at least 1 gene that encodes an Ankyrin repeat protein.

In some embodiments, the recombinant vaccinia virus genome has a deletion of at least 1, 2, or 3 genes that encode an Ankyrin repeat protein. In some embodiments, the genes that encode an Ankyrin repeat protein are, independently, selected from the group consisting of B18R, B20R, and M1L.

In some embodiments, the recombinant vaccinia virus genome has a deletion of at least 1, 2 or 3 genes selected from the group consisting of B15R, B17R, and B14R.

In some embodiments, the recombinant vaccinia virus vector has a deletion of at least 1, 2, 3, 4, 5, 6, 7, or 8 genes selected from the group of ITR genes consisting of B21R, B22R, B23R, B24R, B25R, B26R, B27R, B28R, and B29R. In various embodiments, the recombinant vaccinia virus genome may further include a B8R deletion.

In some embodiments, one or more, or all, of the deletions is a deletion of the entire polynucleotide encoding the corresponding gene. In some embodiments, one or more, or all, of the deletions is a deletion of a portion of the polynucleotide encoding the corresponding gene, such that the deletion is sufficient to render the gene nonfunctional, e.g., upon introduction into a host cell.

In some embodiments, the vector further includes a transgene that encodes a protein that provides improved oncolytic activity, a protein capable of eliciting an immune response for use as a vaccine, a therapeutic polypeptide or a therapeutic nucleic acid. In some embodiments the transgene encodes a protein that provides improved oncolytic activity. In some embodiments the transgene encodes a protein capable of eliciting an immune response for use as a vaccine. In some embodiments the transgene encodes a protein that provides a therapeutic polypeptide. In some embodiments the transgene encodes a therapeutic polypeptide.

In some embodiments, upon contacting a population of mammalian cells (e.g., human cells, such as human cancer cells) with the nucleic acid or the recombinant vaccinia virus vector, the cells exhibit increased syncytia formation relative to a population of mammalian cells of the same type contacted with a form of the vaccinia virus vector that does not include the deletions, as assessed, for instance, by visual inspection using microscopy techniques described herein or known in the art.

In some embodiments, upon contacting a population of mammalian cells (e.g., human cells, such as human cancer cells) with the nucleic acid or the recombinant vaccinia virus vector, the cells exhibit increased spreading of the vaccinia virus vector relative to a population of mammalian cells of the same type contacted with a form of the vaccinia virus vector that does not include the deletions, as assessed, for instance, using plaque-forming assays described herein or known in the art.

In some embodiments, the nucleic acid or the recombinant vaccinia virus vector exerts an increased cytotoxic effect on a population of mammalian cells (e.g., human cells, such as human cancer cells) relative to that of a form of the vaccinia virus vector that does not include the deletions, as assessed, for instance, using cell death assays descried herein or known in the art.

In some embodiments, the mammalian cells are from a cell line selected from the group consisting of U2OS, 293, 293T, Vero, HeLa, A549, BHK, BSC40, CHO, OVCAR-8, 786-0, NCI-H23, U251, SF-295, T-47D, SKMEL2, BT-549, SK-MEL-28, MDA-MB-231, SK-OV-3, MCF7, M14, SF-268, CAKI-1, HPAV, OVCAR-4, HCT15, K-562, and HCT-116.

In another aspect, the invention features a packaging cell line that contains the nucleic acid or the recombinant vaccinia virus vector of any of the aspects or embodiments described herein.

In another aspect, the invention features a method of treating cancer in a mammalian patient by administering a therapeutically effective amount of the nucleic acid or the recombinant vaccinia virus vector to the patient.

In some embodiments, the mammalian patient is a human patient.

In some embodiments, the cancer is selected from the group consisting of leukemia, lymphoma, liver cancer, bone cancer, lung cancer, brain cancer, bladder cancer, gastrointestinal cancer, breast cancer, cardiac cancer, cervical cancer, uterine cancer, head and neck cancer, gallbladder cancer, laryngeal cancer, lip and oral cavity cancer, ocular cancer, melanoma, pancreatic cancer, prostate cancer, colorectal cancer, testicular cancer, and throat cancer.

In some embodiments, the cancer is selected from the group consisting of acute lymphoblastic leukemia (ALL), acute myeloid leukemia (AML), chronic lymphocytic leukemia (CLL), chronic myelogenous leukemia (CML), adrenocortical carcinoma, AIDS-related lymphoma, primary CNS lymphoma, anal cancer, appendix cancer, astrocytoma, atypical teratoid/rhabdoid tumor, basal cell carcinoma, bile duct cancer, extrahepatic cancer, ewing sarcoma family, osteosarcoma and malignant fibrous histiocytoma, central nervous system embryonal tumors, central nervous system germ cell tumors, craniopharyngioma, ependymoma, bronchial tumors, burkitt lymphoma, carcinoid tumor, primary lymphoma, chordoma, chronic myeloproliferative neoplasms, colon cancer, extrahepatic bile duct cancer, ductal carcinoma in situ (DCIS), endometrial cancer, ependymoma, esophageal cancer, esthesioneuroblastoma, extracranial germ cell tumor, extragonadal germ cell tumor, fallopian tube cancer, fibrous histiocytoma of bone, gastrointestinal carcinoid tumor, gastrointestinal stromal tumors (GIST), testicular germ cell tumor, gestational trophoblastic disease, glioma, childhood brain stem glioma, hairy cell leukemia, hepatocellular cancer, langerhans cell histiocytosis, hodgkin lymphoma, hypopharyngeal cancer, islet cell tumors, pancreatic neuroendocrine tumors, wilms tumor and other childhood kidney tumors, langerhans cell histiocytosis, small cell lung cancer, cutaneous T cell lymphoma, intraocular melanoma, merkel cell carcinoma, mesothelioma, metastatic squamous neck cancer, midline tract carcinoma, multiple endocrine neoplasia syndromes, multiple myeloma/plasma cell neoplasm, myelodysplastic syndromes, nasal cavity and paranasal sinus cancer, nasopharyngeal cancer, neuroblastoma, non-hodgkin lymphoma (NHL), non-small cell lung cancer (NSCLC), epithelial ovarian cancer, germ cell ovarian cancer, low malignant potential ovarian cancer, pancreatic neuroendocrine tumors, papillomatosis, paraganglioma, paranasal sinus and nasal cavity cancer, parathyroid cancer, penile cancer, pharyngeal cancer, pheochromocytoma, pituitary tumor, pleuropulmonary blastoma, primary peritoneal cancer, rectal cancer, retinoblastoma, rhabdomyosarcoma, salivary gland cancer, kaposi sarcoma, rhabdomyosarcoma, sezary syndrome, small intestine cancer, soft tissue sarcoma, throat cancer, thymoma and thymic carcinoma, thyroid cancer, transitional cell cancer of the renal pelvis and ureter, urethral cancer, endometrial uterine cancer, uterine sarcoma, vaginal cancer, vulvar cancer, and Waldenström macroglobulinemia.

In another aspect, the invention features a kit containing the nucleic acid or vector of any of the aspects or embodiments described herein and a package insert instructing a user of the kit to express the nucleic acid or vector in a host cell.

In another aspect, the invention features a kit containing the nucleic acid or recombinant vaccinia virus vector of any of the aspects or embodiments described herein and a package insert instructing a user to administer a therapeutically effective amount of the nucleic acid or recombinant vaccinia virus vector to a mammalian patient (e.g., a human patient) having cancer, thereby treating the cancer.

### Definitions

As used herein, the term **"about"** refers to a value that is no more than 10% above or below the value being described. For example, the term "about 5 nM" indicates a range of from 4.5 nM to 5.5 nM.

As used herein, the terms **"delete," "deletion,"** and the like refer to modifications to a gene or a regulatory element associated therewith or operatively linked thereto (e.g., a transcription factor-binding site, such as a promoter or enhancer element) that remove the gene or otherwise render the gene nonfunctional. Exemplary deletions, as described herein, include the removal of the entirety of a nucleic acid encoding a gene of interest, from the start codon to the stop codon of the target gene. Other examples of deletions as described herein include the removal of a portion of the nucleic acid encoding the target gene (e.g., one or more codons, or a portion thereof, such as a single nucleotide deletion) such that, upon expression of the partially-deleted target gene, the product (e.g., RNA transcript, protein product, or regulatory RNA, such as a miRNA) is nonfunctional or less functional then a wild-type form of the target gene. Exemplary deletions as described herein include the removal of all or a portion of the regulatory element(s) associated with a gene of interest, such as all or a portion of the promoter and/or enhancer nucleic acids that regulate expression of the target gene.

As used herein, the term **"endogenous"** describes a molecule (e.g., a polypeptide, nucleic acid, or cofactor) that is found naturally in a particular organism (e.g., a human) or in a particular location within an organism (e.g., an organ, a tissue, or a cell, such as a human cell).

As used herein, the term **"percent(%) sequence identity"** refers to the percentage of amino acid (or nucleic acid) residues of a candidate sequence that are identical to the amino acid (or nucleic acid) residues of a reference sequence after aligning the sequences and introducing gaps, if necessary, to achieve the maximum percent sequence identity (e.g., gaps can be introduced in one or both of the candidate and reference sequences for optimal alignment and non-homologous sequences can be disregarded for comparison purposes). Alignment for purposes of determining percent sequence identity can be achieved in various ways that are within the skill in the art, for instance, using publicly available computer software, such as BLAST, ALIGN, or Megalign (ONASTAR) software. Those skilled in the art can determine appropriate parameters for measuring alignment, including any algorithms needed to achieve maximal alignment over the full length of the sequences being compared. For example, a reference sequence aligned for comparison with a candidate sequence may show that the candidate sequence exhibits from 50% to 100% sequence identity across the full length of the candidate sequence or a selected portion of contiguous amino acid (or nucleic acid) residues of the candidate sequence. The length of the candidate sequence aligned for comparison purposes may be, for example, at least 30%, (e.g., 30%, 40, 50%, 60%, 70%, 80%, 90%, or 100%) of the length of the reference sequence. When a 5 position in the candidate sequence is occupied by the same amino acid residue as the corresponding position in the reference sequence, then the molecules are identical at that position.

As used herein, the terms **"subject"** and **"patient"** refer to an organism that receives treatment for a particular disease or condition as described herein (such as cancer or an infectious disease). Examples of subjects and patients include mammals, such as humans, receiving treatment for diseases or conditions, for example, cell proliferation disorders, such as cancer.

As used herein, the terms **"treat"** or **"treatment"** refer to therapeutic treatment, in which the object is to prevent or slow down (lessen) an undesired physiological change or disorder, such as the progression of a cell proliferation disorder, such as cancer. Beneficial or desired clinical results include, but are not limited to, alleviation of symptoms, diminishment of extent of disease, stabilized (i.e., not worsening) state of disease, delay or slowing of disease progression, amelioration or palliation of the disease state, and remission (whether partial or total), whether detectable or undetectable. Those in need of treatment include those already with the condition or disorder, as well as those prone to have the condition or disorder or those in which the condition or disorder is to be prevented.

As used herein, the term **"vector"** refers to a nucleic acid vector, e.g., a DNA vector, such as a plasmid, a RNA vector, virus or other suitable replicon (e.g., viral vector). A variety of vectors have been developed for the delivery of polynucleotides encoding exogenous proteins into a prokaryotic or eukaryotic cell. Examples of such expression vectors are disclosed in, e.g., WO 1994/1 1026; incorporated herein by reference. Expression vectors of the invention may contain one or more additional sequence elements used for the expression of proteins and/or the integration of these polynucleotide sequences into the genome of a host cell, such as a mammalian cell (e.g., a human cell). Exemplary vectors that can be used for the expression of antibodies and antibody fragments described herein include plasmids that contain regulatory sequences, such as promoter and enhancer regions, which direct gene transcription. Vectors may contain nucleic acids that modulate the rate of translation of a target gene or that improve the stability or nuclear export of the mRNA that results from gene transcription. These sequence elements may include, e.g., 5' and 3' untranslated regions, an internal ribosomal entry site (IRES), and polyadenylation signal site in order to direct efficient transcription of the gene carried on the expression vector. The vectors described herein may also contain a polynucleotide encoding a marker for selection of cells that contain such a vector. Examples of a suitable marker include genes that encode resistance to antibiotics, such as ampicillin, chloramphenicol, kanamycin, or nourseothricin.

### Gene Definitions

As used herein, **"C2L"** refers to a vaccinia virus gene, such as a gene that encodes a kelch-like protein. Non-limiting examples of protein sequences encoding the C2L gene are listed in tables 31-35 below. The term "C2L" may also include fragments or variants of the proteins listed in the tables below, or homologous genes from another vaccinia virus strain.

As used herein, "**C1L**" refers to a vaccinia virus gene. Non-limiting examples of protein sequences encoding the C2L gene are listed in tables 31-35 below. The term "C1L" may also include fragments or variants of the proteins listed in the tables below, or homologous genes from another vaccinia virus strain.

As used herein, **"NIL"** refers to a vaccinia virus gene, such as a gene that encodes a BCL-2 inhibitor. Non-limiting examples of protein sequences encoding the N1L gene are listed in tables 31-35 below. The term "NIL" may also include fragments or variants of the proteins listed in the tables below, or homologous genes from another vaccinia virus strain.

As used herein, **"N2L"** refers to a vaccinia virus gene, such as a gene that encodes a TLR signaling inhibitor. Non-limiting examples of protein sequences encoding the N2L gene are listed in tables 31-35 below. The term "N2L" may also include fragments or variants of the proteins listed in the tables below, or homologous genes from another vaccinia virus strain.

As used herein, **"MIL"** refers to a vaccinia virus gene, such as a gene that encodes an Ankyrin repeat protein. Non-limiting examples of protein sequences encoding the M1L gene are listed in tables 31-35 below. The term "MIL" may also include fragments or variants of the proteins listed in the tables below, or homologous genes from another vaccinia virus strain.

As used herein, **"M2L"** refers to a vaccinia virus gene, such as a gene that encodes an NF-κB inhibitor. Non-limiting examples of protein sequences encoding the M2L gene are listed in tables 31-35 below. The term "M2L" may also include fragments or variants of the proteins listed in the tables below, or homologous genes from another vaccinia virus strain.

As used herein, "**K1L**" refers to a vaccinia virus gene, such as a gene that encodes an NF-κB inhibitor. Non-limiting examples of protein sequences encoding the K1L gene are listed in tables 31-35 below. The term "K1L" may also include fragments or variants of the proteins listed in the tables below, or homologous genes from another vaccinia virus strain.

As used herein, **"K2L"** refers to a vaccinia virus gene, such as a gene that encodes an Ankyrin repeat protein. Non-limiting examples of protein sequences encoding the K2L gene are listed in tables 31-35 below. The term "K2L" may also include fragments or variants of the proteins listed in the tables below, or homologous genes from another vaccinia virus strain.

As used herein, **"K3L"** refers to a vaccinia virus gene, such as a gene that encodes a PKR inhibitor. Non-limiting examples of protein sequences encoding the K3L gene are listed in tables 31-35 below. The term "K3L" may also include fragments or variants of the proteins listed in the tables below, or homologous genes from another vaccinia virus strain.

As used herein, **"K4L"** refers to a vaccinia virus gene, such as a gene that encodes a phospholipase-D. Non-limiting examples of protein sequences encoding the K4L gene are listed in tables 31-35 below. The term "K4L" may also include fragments or variants of the proteins listed in the tables below, or homologous genes from another vaccinia virus strain.

As used herein, **"K5L"** refers to a vaccinia virus gene, such as a gene that encodes a monoglyceride lipase. Non-limiting examples of protein sequences encoding the K5L gene are listed in tables 31-35 below. The term "K5L" may also include fragments or variants of the proteins listed in the tables below, or homologous genes from another vaccinia virus strain.

As used herein, **"K6L"** refers to a vaccinia virus gene, such as a gene that encodes a monoglyceride lipase. Non-limiting examples of protein sequences encoding the K6L gene are listed in tables 31-35 below. The term "K6L" may also include fragments or variants of the proteins listed in the tables below, or homologous genes from another vaccinia virus strain.

As used herein, **"K7R"** refers to a vaccinia virus gene, such as a gene that encodes an NF-κB inhibitor. Non-limiting examples of protein sequences encoding the K7R gene are listed in tables 31-35 below. The term "K7R" may also include fragments or variants of the proteins listed in the tables below, or homologous genes from another vaccinia virus strain..

As used herein, "**F1L**" refers to a vaccinia virus gene, such as a gene that encodes a caspase-9 inhibitor. Non-limiting examples of protein sequences encoding the F1L gene are listed in tables 31-35 below. The term "F1L" may also include fragments or variants of the proteins listed in the tables below, or homologous genes from another vaccinia virus strain.

As used herein, **"F2L"** refers to a vaccinia virus gene, such as a gene that encodes a dUTPase. Non-limiting examples of protein sequences encoding the F2L gene are listed in tables 31-35 below. The term "F2L" may also include fragments or variants of the proteins listed in the tables below, or homologous genes from another vaccinia virus strain.

As used herein, **"F3L"** refers to a vaccinia virus gene, such as a gene that encodes a kelch-like protein. Non-limiting examples of protein sequences encoding the F3L gene are listed in tables 31-35 below. The term "F1L" may also include fragments or variants of the proteins listed in the tables below, or homologous genes from another vaccinia virus strain.

As used herein, **"B14R"** refers to a vaccinia virus gene. Non-limiting examples of protein sequences encoding the B14R gene are listed in tables 36-40 below. The term "B14R" may also include fragments or variants of the proteins listed in the tables below, or homologous genes from another vaccinia virus strain.

As used herein, **"B15R"** refers to a vaccinia virus gene. Non-limiting examples of protein sequences encoding the B15R gene are listed in tables 36-40 below. The term "B15R" may also include fragments or variants of the proteins listed in the tables below, or homologous genes from another vaccinia virus strain.

As used herein, **"B16R"** refers to a vaccinia virus gene, such as a gene that encodes a IL-1-beta inhibitor. Non-limiting examples of protein sequences encoding the B16R gene are listed in tables 31-35 below. The term "B16R" may also include fragments or variants of the proteins listed in the tables below, or homologous genes from another vaccinia virus strain.

As used herein, **"B17L"** refers to a vaccinia virus gene. Non-limiting examples of protein sequences encoding the B17L gene are listed in tables 36-40 below. The term "B17L" may also include fragments or variants of the proteins listed in the tables below, or homologous genes from another vaccinia virus strain.

As used herein, **"B18R"** refers to a vaccinia virus gene, such as a gene that encodes an Ankyrin repeat protein. Non-limiting examples of protein sequences encoding the B18R gene are listed in tables 36-40 below. The term "B18R" may also include fragments or variants of the proteins listed in the tables below, or homologous genes from another vaccinia virus strain.

As used herein, **"B19R"** refers to a vaccinia virus gene, such as a gene that encodes a IFN-alpha-beta-receptor-like secreted glycoprotein. Non-limiting examples of protein sequences encoding the B19R gene are listed in tables 36-40 below. The term "B19R" may also include fragments or variants of the proteins listed in the tables below, or homologous genes from another vaccinia virus strain.

As used herein, **"B20R"** refers to a vaccinia virus gene, such as a gene that encodes an Ankyrin repeat protein. Non-limiting examples of protein sequences encoding the B20R gene are listed in tables 36-40 below. The term "B20R" may also include fragments or variants of the proteins listed in the tables below, or homologous genes from another vaccinia virus strain.

As used herein, **"B8R"** refers to a vaccinia virus gene, such as a gene that encodes a secreted protein with homology to the gamma interferon (IFN-γ). A nonlimiting example of a protein sequence encoded by an exemplary B8R gene in a Copenhagen strain of the vaccinia virus is given in UniProtKB database entry P21004 and is reproduced below: 
MRYIIILAVLFINSIHAKITSYKFESVNFDSKIEWTGDGLYNISLKNYGIKTWQTMYTN
VPEGTYDISAFPKNDFVSFWVKFEQGDYKVEEYCTGLCVEVKIGPPTVTLTEYDDHI
NLYIEHPYATRGSKKIPIYKRGDMCDIYLLYTANFTFGDSEEPVTYDIDDYDCTSTGC
SIDFATTEKVCVTAQGATEGFLEKITPWSSEVCLTPKKNVYTCAIRKEDVPNFKD
KMARVIKRKFNKQSQSYLTKFLGSTSNDVTTFLSMLNLTKYS
 The term "B8R" may also include fragments or variants of the proteins listed above, or homologous genes from another vaccinia virus strain. Variants include without limitation those sequences having 85 percent or greater identity to the sequences disclosed herein.

### Brief Description of the Figures

**FIG. 1** shows the phylogenetic analysis of 59 poxvirus strains, including the Vaccinia virus virus strains.
**FIG. 2** shows the abundance of different viral strains after passaging 5 Vaccinia viruses in different tumor types.
**FIG. 3** shows the ability to replicate in various different patient tumor cores of Vaccinia wild-type strains.
**FIG. 4** shows plaque size measurements of different Vaccinia wild-type strains.
**FIG. 5A** shows the number of TTAA sites across 1kb regions in Vaccinia Copenhagen genome.
**FIG. 5B** shows the frequency of Transposon Insertions across Vaccinia Copenhagen genome. Each dot represents a transposon knockout of a particular gene. The position of the dot on the y-axis is determined by the frequency of the knockout.
**FIG. 5C** shows Poxvirus gene conservation in 59 viruses. Higher conservation indicates the gene is present in a larger amount of species.
**FIG. 6** shows the frequency of various transposon knockouts after passaging in permissive cancer cells.

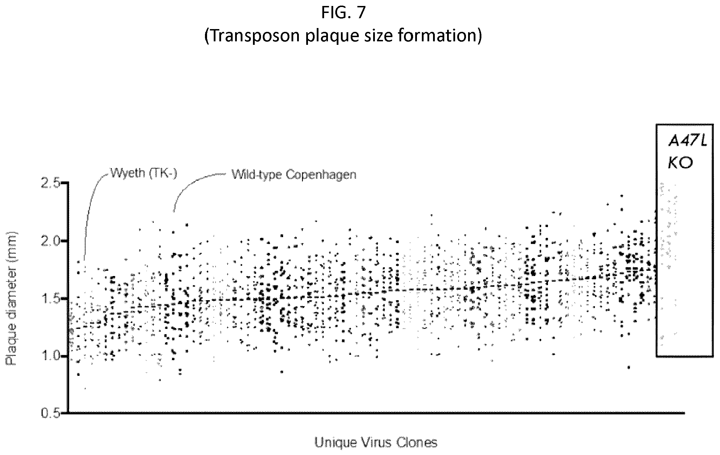

**FIG. 7** shows plaque size measurements of purified transposons.
**FIG. 8** shows the genomic structure of a 5p deletion (CopMD5p) and a 3p deletion (CopMD3p). Both CopMD5p and CopMD3p were crossed to generate CopMD5p3p.
**FIG. 9** shows a heatmap showing cancer cell death following infection with either Copenhagen or CopMD5p3p at various doses.
**FIG. 10** shows the growth curves of Copenhagen and CopMD5p3p replication in 4 different cancer cell lines.
**FIG. 11** shows the ability of Copenhagen and CopMD5p3p to replicate in patient ex vivo samples as shown by tittering.
**FIG. 12** shows that the modified CopMD5p3p virus forms different plaques than the parental virus. CopMD5p3p plaques are much clearer in the middle and we can see syncytia (cell fusion).
**FIG. 13** shows CopMD5p3p induces syncytia (cell fusion) in 786-O cells.
**FIG. 14** shows that CopMD5p3p is able to control tumour growth similarly to Copenhagen wild-type but does not cause weight loss.
**FIG. 15** shows that CopMD5p3p does not cause pox lesion formation when compared to two other Vaccinia strains (Copenhagen and Wyeth) harboring the oncolytic knockout of thymidine kinase.
**FIG. 16** shows the IVIS bio-distribution of Vaccinia after systemic administration in nude CD-1 mice. Luciferase encoding CopMD5p3p (TK KO) is tumor specific and does not replicate in off target tissues.
**FIG. 17** shows the bio-distribution of Vaccinia after systemic administration. CopMD5p3p replicates similarly to other oncolytic Vaccinia in the tumour but replicates less in off target tissues/organs.
**FIG. 18** shows the immunogenicity of Vaccinia in Human PBMCs. The ability of CopMD5p3p to induce human innate immune cell activation is stronger than that of wild-type Copenhagen.
**FIG. 19** shows the immunogenicity of Vaccinia in Mouse Splenocytes. The ability of CopMD5p3p to induce mouse innate immune cell activation is stronger than that of Copenhagen.
**FIG. 20** shows the immunogenicity of Vaccinia in Human cells. The ability of CopMD5p3p to activate NF-kB immune transcription factor is stronger than that of Copenhagen or VVdd but similar to that of MG-1.

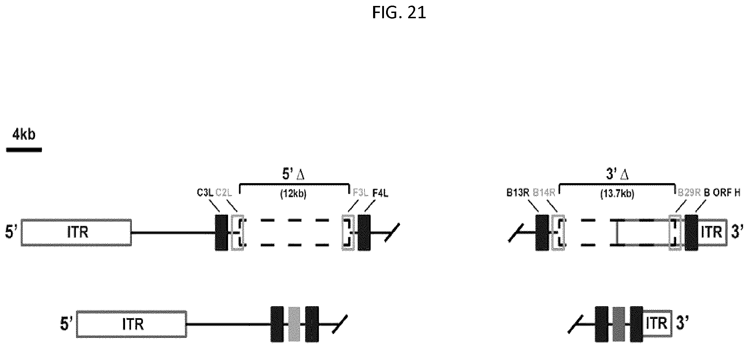

**FIG. 21** shows a schematic representation of the homologous recombination targeting strategy employed to generate denovo 5p (left) and 3p (right) major deletions in various vaccinia strains.

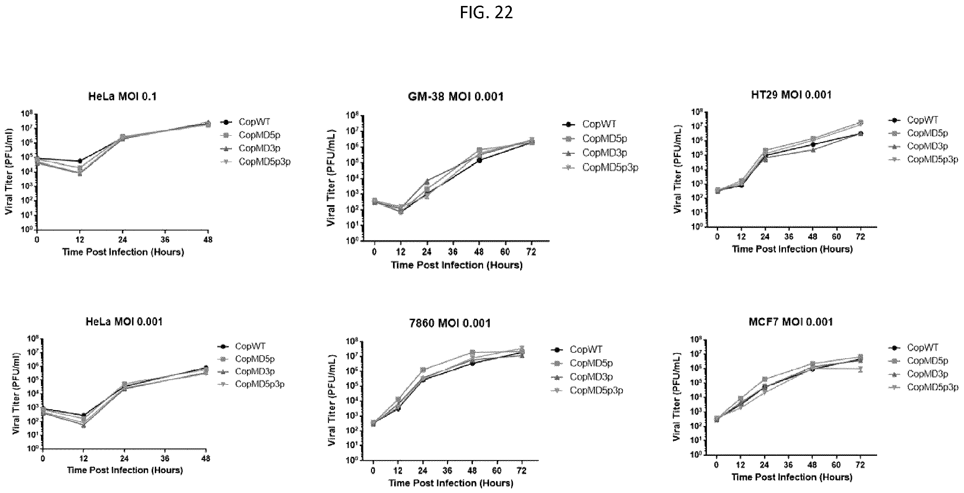

**FIG. 22** shows the ability of wild-type Copenhagen vaccinia virus and several modified Copenhagen vaccinia virions to proliferate in various cell lines.

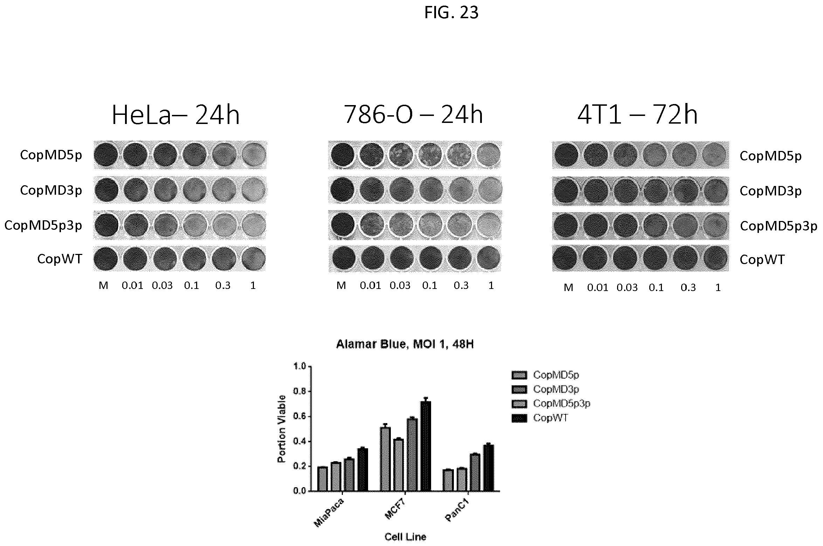

**FIG. 23** shows the cytotoxic effects of wild-type Copenhagen vaccinia virus and several modified Copenhagen vaccinia virions on various cell lines, as assessed by crystal violet (upper panel) and an Alamar Blue assay (lower panel). The order of strains listed for each cell line along the x-axis of the chart shown in the lower panel is as follows: from left to right, CopMD5p, CopMD5p3p, CopMD3p, and CopWT.

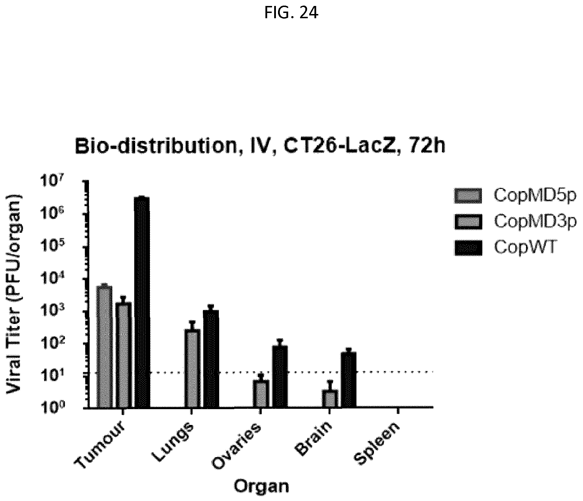

**FIG. 24** shows the distribution of wild-type Copenhagen vaccinia virus and several modified Copenhagen vaccinia virions upon administration to mice.
**FIG. 25** shows the ability of wild-type Copenhagen vaccinia virus and several modified Copenhagen vaccinia virions to activate Natural Killer (NK) cells and promote anti-tumor immunity.
**FIG. 26** shows the ability of wild-type Copenhagen vaccinia virus and several modified Copenhagen vaccinia virions to enhance NK cell-mediated degranulation against HT29 cells, a measure of NK cell activity and anti-tumor immunity.
**FIG. 27** shows the ability of wild-type Copenhagen vaccinia virus and several modified Copenhagen vaccinia virions to prime T-cells to initiate an anti-tumor immune response.
**FIG. 28** shows the ability of wild-type Copenhagen vaccinia virus and several modified Copenhagen vaccinia virions to spread to distant locations from the initial point of infection.
**FIG. 29** shows the ability of wild-type Copenhagen vaccinia virus and several modified Copenhagen vaccinia virions to form plaques, a measure of viral proliferation.
**FIG. 30** shows the ability of wild-type Copenhagen vaccinia virus and several modified Copenhagen vaccinia virions to form plaques in 786-O cells.
**FIG. 31** shows the percentage of genes deleted in CopMD5p3p in various poxvirus genomes.
**FIG. 32** shows infection of normal versus cancer cell lines of SKV-B8R+ virus.
**FIG. 33** shows SKV-B8R+ does not impair interferon signaling.
**FIG. 34** shows SKV (CopMD5p3-B8R-) has similar efficacy in tumour control compared to SKV-B8R+.
**FIG. 35** shows major double deletions engineered in various vaccinia strains enhance cancer cell killing in vitro.
**FIG. 36** shows the phenotypic characterization of HeLa cells infected with various vaccinia strains.
**FIG. 37** shows 5p3p vaccinia strains do not induce weight loss compared to wildtype strains.
**FIG. 38** shows 5p3p vaccinia strains do not induce pox lesions compared to wildtype strains.

### Detailed Description

The present invention features genetically modified Copenhagen-derived vaccinia viruses, as well as the use of the same for the treatment of various cancers. The invention is based in part on the surprising discovery that vaccinia viruses, such as Copenhagen, exhibit markedly improved oncolytic activity, replication in tumors, infectivity, immune evasion, tumor persistence, capacity for incorporation of exogenous DNA sequences, and amenability for large scale manufacturing when the viruses are engineered to contain deletions in one or more, or all, of the C2L, C1L, N1L, N2L, MIL, M2L, K1L, K2L, K3L, K4L, K5L, K6L, K7R, F1L, F2L, F3L, B14R, B15R, B16R, B17L, B18R, B19R, B20R, K ORF A, K ORF B, B ORF E, B ORF F, B ORF G, B21R, B22R, B23R, B24R, B25R, B26R, B27R, B28R, and B29R. In various embodiments of the invention, the modified vaccinia viruses contain a deletion of the B8R gene. While inactive in mice, the B8R gene neutralizes antiviral activity of human IFN-γ. In various embodiments, at least one transgene is subsequently inserted into locus of the B8R gene (now deleted) through a homologous recombination targeting strategy.

The vaccinia viruses described herein can be administered to a patient, such as a mammalian patient (e.g., a human patient) to treat a variety of cell proliferation disorders, including a wide range of cancers. The sections that follow describe vaccinia viruses and genetic modifications thereto, as well as methods of producing and propagating genetically modified vaccinia viruses and techniques for administering the same to a patient.

### Vaccinia Virus

Vaccinia virus is a member of the orthopoxvirus or Poxviridae family, the Chordopoxyirinae subfamily, and the Orthopoxvirus genus. Orthopoxvirus is relatively more homogeneous than other members of the Chordopoxyirinae subfamily and includes 11 distinct but closely related species, which includes vaccinia virus, variola virus (causative agent of smallpox), cowpox virus, buffalopox virus, monkeypox virus, mousepox virus and horsepox virus species as well as others (see Moss, 1996). Certain embodiments of the invention, as described herein, may be extended to other members of Orthopoxvirus genus as well as the Parapoxvirus, Avipoxvirus, Capripoxvirus, Leporipoxvirus, Suipoxvirus, Molluscipoxvirus, and Yatapoxvirus genus. A genus of orthopoxvirus family is generally defined by serological means including neutralization and cross-reactivity in laboratory animals. Various members of the Orthopoxvirus genus, as well as other members of the Chordovirinae subfamily utilize immunomodulatory molecules, examples of which are provided herein, to counteract the immune responses of a host organism.

Vaccinia virus is a large, complex enveloped virus having a linear double-stranded DNA genome of about 190K by and encoding for approximately 250 genes. Vaccinia is well-known for its role as a vaccine that eradicated smallpox. Post-eradication of smallpox, scientists have been exploring the use of vaccinia as a tool for delivering genes into biological tissues (gene therapy and genetic engineering). Vaccinia virus is unique among DNA viruses as it replicates only in the cytoplasm of the host cell. Therefore, the large genome is required to code for various enzymes and proteins needed for viral DNA replication. During replication, vaccinia produces several infectious forms which differ in their outer membranes: the intracellular mature virion (IMV), the intracellular enveloped virion (IEV), the cell-associated enveloped virion (CEV), and the extracellular enveloped virion (EEV). IMV is the most abundant infectious form and is thought to be responsible for spread between hosts. On the other hand, the CEV is believed to play a role in cell-to-cell spread and the EEV is thought to be important for long range dissemination within the host organism.

Vaccinia virus is closely related to the virus that causes cowpox. The precise origin of vaccinia is unknown, but the most common view is that vaccinia virus, cowpox virus, and variola virus (the causative agent for smallpox) were all derived from a common ancestral virus. There is also speculation that vaccinia virus was originally isolated from horses. A vaccinia virus infection is mild and typically asymptomatic in healthy individuals, but it may cause a mild rash and fever, with an extremely low rate of fatality. An immune response generated against a vaccinia virus infection protects that person against a lethal smallpox infection. For this reason, vaccinia virus was used as a live-virus vaccine against smallpox. The vaccinia virus vaccine is safe because it does not contain the smallpox virus, but occasionally certain complications and/or vaccine adverse effects may arise, especially if the vaccine is immunocompromised.

Exemplary strains of the vaccinia virus include the Copenhagen-derived vaccina virus.

### Thymidine Kinase Mutants

Several current clinical studies testing vaccinia virus as an oncolytic virus harbor deletions in the viral Thymidine Kinase (TK) gene. This deletion attenuates the virus, rendering the virus dependent upon the activity of cellular thymidine kinase for DNA replication and, thus, viral propagation. Cellular thymidine kinase is expressed at a low level in most normal tissues and at elevated levels in many cancer cells. Through metabolic targeting, TK- viruses can grow in cells that have a high metabolic rate (e.g., healthy cells or tumor cells) and will not grow well in cells that have low levels of thymidine kinase. Since there exist quiescent tumour cells (e.g., cancer stem cells), TK- viruses are likely compromised in their ability to kill this population of cancer cells just as chemotherapy is largely ineffective. The modified viral vectors described in this disclosure retains virus synthetic machinery (including TK) and may propagate in quiescent cancer cells. The viral modifications of this disclosure may allow the virus to be highly selective without deleting TK or other DNA metabolizing enzymes (e.g., ribonucleotide reductase) and could be more effective in tumors with a low metabolic rate.

### Virus Propagation

The present invention features vaccinia viruses, including those constructed with one or more gene deletions compared to wild-type, such that the virus exhibits desirable properties for use against cancer cells, while being less toxic or non-toxic to non-cancer cells. This section summarizes various protocols, by way of example, for producing recombinant vaccinia viruses described herein, such as methods for generating mutated viruses through the use of recombinant DNA technology.

For example, to generate mutations in the vaccinia virus genome, native and modified polypeptides may be encoded by a nucleic acid molecule comprised in a vector. Vectors include plasmids, cosmids, viruses (bacteriophage, animal viruses, and plant viruses), and artificial chromosomes (e.g., YACs). One of skill in the art would be well equipped to construct a vector through standard recombinant techniques, which are described in Sambrook et al., (1989) and Ausubel et al., 1994, both incorporated herein by reference. In addition to encoding a modified polypeptide such as modified gelonin, a vector may encode non-modified polypeptide sequences such as a tag or targeting molecule.

In order to propagate a vector in a host cell, it may contain one or more origins of replication sites (often termed "ori"), which is a specific nucleic acid sequence at which replication is initiated. Alternatively an autonomously replicating sequence (ARS) can be employed if the host cell is yeast.

In the context of expressing a heterologous nucleic acid sequence, "host cell" refers to a prokaryotic or eukaryotic cell, and it includes any transformable organisms that is capable of replicating a vector and/or expressing a heterologous gene encoded by a vector. A host cell can, and has been, used as a recipient for vectors or viruses (which does not qualify as a vector if it expresses no exogenous polypeptides). A host cell may be "transfected" or "transformed," which refers to a process by which exogenous nucleic acid, such as a modified protein-encoding sequence, is transferred or introduced into the host cell. A transformed cell includes the primary subject cell and its progeny. Host cells may be derived from prokaryotes or eukaryotes, including yeast cells, insect cells, and mammalian cells, depending upon whether the desired result is replication of the vector or expression of part or all of the vector-encoded nucleic acid sequences. Numerous cell lines and cultures are available for use as a host cell, and they can be obtained through the American Type Culture Collection (ATCC), which is an organization that serves as an archive for living cultures and genetic materials (www.atcc.org). An appropriate host can be determined by one of skill in the art based on the vector backbone and the desired result. A plasmid or cosmid, for example, can be introduced into a prokaryote host cell for replication of many vectors. Bacterial cells used as host cells for vector replication and/or expression include DH5α, JM109, and KCB, as well as a number of commercially available bacterial hosts such as SURE^{®} Competent Cells and SOLOPACK^{™} Gold Cells (STRATAGENE^{®}, La Jolla, Calif.). Alternatively, bacterial cells such as E. coli LE392 could be used as host cells for phage viruses. Appropriate yeast cells include Saccharomyces cerevisiae, Saccharomyces pombe, and Pichia pastoris. Examples of eukaryotic host cells for replication and/or expression of a vector include HeLa, NIH3T3, Jurkat, 293, Cos, CHO, Saos, and PC12. Many host cells from various cell types and organisms are available and would be known to one of skill in the art. Similarly, a viral vector may be used in conjunction with either a eukaryotic or prokaryotic host cell, particularly one that is permissive for replication or expression of the vector. Some vectors may employ control sequences that allow it to be replicated and/or expressed in both prokaryotic and eukaryotic cells. One of skill in the art would further understand the conditions under which to incubate all of the above described host cells to maintain them and to permit replication of a vector. Also understood and known are techniques and conditions that would allow large-scale production of vectors, as well as production of the nucleic acids encoded by vectors and their cognate polypeptides, proteins, or peptides.

### Genetic Modifications to the Vaccinia virus Genome

### Methods of Genetic Modification.

Methods for the insertion or deletion of nucleic acids from a target genome include those described herein and known in the art. One such method that can be used for incorporating polynucleotides encoding target genes into a target genome involves the use of transposons. Transposons are polynucleotides that encode transposase enzymes and contain a polynucleotide sequence or gene of interest flanked by 5' and 3' excision sites. Once a transposon has been delivered to a target nucleic acid (e.g., in a host cell), expression of the transposase gene commences and results in active enzymes that cleave the gene of interest from the transposon. This activity is mediated by the site-specific recognition of transposon excision sites by the transposase. In certain cases, these excision sites may be terminal repeats or inverted terminal repeats. Once excised from the transposon, the gene of interest can be integrated into the target genome by transposase-catalyzed cleavage of similar excision sites that exist within the nuclear genome of the cell. This allows the gene of interest to be inserted into the cleaved nuclear DNA at the complementary excision sites, and subsequent covalent ligation of the phosphodiester bonds that join the gene of interest to the DNA of the target genome completes the incorporation process. In certain cases, the transposon may be a retrotransposon, such that the gene encoding the target gene is first transcribed to an RNA product and then reverse-transcribed to DNA before incorporation in the mammalian cell genome. Transposon systems include the piggybac transposon (described in detail in, e.g., WO 2010/085699) and the sleeping beauty transposon (described in detail in, e.g., US2005/0112764), the disclosures of each of which are incorporated herein by reference.

Additional methods for nucleic acid delivery to effect expression of compositions of the present invention are believed to include virtually any method by which a nucleic acid (e.g., DNA, including viral and non-viral vectors) can be introduced into an organelle, a cell, a tissue or an organism, as described herein or as would be known to one of ordinary skill in the art. Such methods include, but are not limited to, direct delivery of DNA such as by injection (U.S. Pat. Nos. 5,994,624, 5,981,274, 5,945,100, 5,780,448, 5,736,524, 5,702,932, 5,656,610, 5,589,466 and 5,580,859, each incorporated herein by reference), including microinjection (Harland and Weintraub, 1985; U.S. Pat. No. 5,789,215, incorporated herein by reference); by electroporation (U.S. Pat. No. 5,384,253, incorporated herein by reference); by calcium phosphate precipitation (Graham and Van Der Eb, 1973; Chen and Okayama, 1987; Rippe et al., 1990); by using DEAE-dextran followed by polyethylene glycol (Gopal, 1985); by direct sonic loading (Fechheimer et al., 1987); by liposome mediated transfection (Nicolau and Sene, 1982; Fraley et al., 1979; Nicolau et al., 1987; Wong et al., 1980; Kaneda et al., 1989; Kato et al., 1991); by microprojectile bombardment (PCT Application Nos. WO 94/09699 and 95/06128; U.S. Pat. Nos. 5,610,042; 5,322,783, 5,563,055, 5,550,318, 5,538,877 and 5,538,880, and each incorporated herein by reference); by agitation with silicon carbide fibers (Kaeppler et al., 1990; U.S. Pat. Nos. 5,302,523 and 5,464,765, each incorporated herein by reference); by Agrobacterium-mediated transformation (U.S. Pat. Nos. 5,591,616 and 5,563,055, each incorporated herein by reference); or by PEG-mediated transformation of protoplasts (Omirulleh et al., 1993; U.S. Pat. Nos. 4,684,611 and 4,952,500, each incorporated herein by reference); by desiccation/inhibition-mediated DNA uptake (Potrykus et al., 1985). Through the application of techniques such as these, organelle(s), cell(s), tissue(s) or organism(s) may be stably or transiently transformed. *CopMD5p, CopMD3p, and CopMD5p3p Deletions.*

In various embodiments, various genes are deleted to enhance the oncolytic activity of the vaccinia virus. Most of the deletions described herein are either involved in blocking a host response to viral infection or otherwise have an unknown function. In various embodiments, at least one of the genes depicted in Table 1 are deleted from the recombinant vaccinia virus genome. In various embodiments, at least 2, 3, 4, 5, 6, 7, 8, 9, 10, 11, 12, 13, 14, 15, 16, 17, 18, 19, 20, 21, 22, 23, 24, 25, 26, 27, 28, 29, 30 or 31 of the genes depicted in Table 2 are deleted from the recombinant vaccinia genome. In various embodiments, all of the genes depicted in Table 2 are deleted from the recombinant vaccinia virus genome. Three exemplary embodiments of the present invention, CopMD5p, CopMD3p and CopMD5p3p, are described herein. Depicted in Table 1 below are clusters of deleted genes and their function in CopMD5p, CopMD3p, and CopMD5p3p virus. In various embodiments, where two copies of an ITR exist, only the right ITR of the genome is deleted and the left ITR remains intact. Deletions were confirmed by whole genome sequencing.

**Table 1: Deleted genes in Vaccinia viruses**

| Name | Category | Function | Virus Deletions | |
|---|---|---|---|---|
| C2L | Host interaction | Inhibits NFkB | CopMD5p | CopMD5p3p |
| C1L | Unknown | Unknown | | |
| N1L | Host interaction | Inhibits NFkB and Apoptosis | | |
| N2L | Host interaction | Inhibits IRF3 | | |
| M1L | Unknown | Unknown | | |
| M2L | Host interaction | Inhibits NFkB and Apoptosis | | |
| K1L | Host interaction | Inhibits PKR and NF-kB | | |
| K2L | Host interaction | Prevents cell fusion | | |
| K3L | Host interaction | Inhibits PKR | | |
| K4L | DNA replication | DNA modifying nuclease | | |
| K5L | Pseudogene | Pseudogene | | |
| K6L | Pseudogene | Pseudogene | | |
| K7R | Host interaction | Inhibits NFkB and IRF3 | | |
| F1L | Host interaction | Inhibits Apoptosis | | |
| F2L | DNA replication | Deoxyuridine triphosphatase | | |
| F3L | Host interaction | Virulence factor | | |
| B14R | Pseudogene | Pseudogene | CopMD3p | |
| B15R | Unknown | Unknown | | |
| B16R | Host interaction | IL-1-beta-inhibitor | | |
| B17L | Unknown | Unknown | | |
| B18R | Unknown | Ankyrin-like | | |
| B19R | Host interaction | Secreted IFNa sequestor | | |
| B20R | Unknown | Ankyrin-like | | |
| B21R-ITR* | Unknown | Unknown | | |
| B22R-ITR* | Unknown | Unknown | | |
| B23R-ITR* | Unknown | Unknown | | |
| B24R-ITR* | Unknown | Unknown | | |
| B25R-ITR* | Unknown | Unknown | | |
| B26R-ITR* | Unknown | Unknown | | |
| B27R-ITR* | Unknown | Unknown | | |
| B28R-ITR* | Pseudogene | TNF-a receptor | | |
| B29R-ITR* | Host interaction | Secreted CC-chemokine sequestor | | |

### B8R Gene Deletions.

In various embodiments, the vaccinia viruses are further genetically modified to contain deletions in the B8R gene. The vaccinia virus B8R gene encodes a secreted protein with homology to gamma interferon receptor (IFN-γ). In vitro, the B8R protein binds to and neutralizes the antiviral activity of several species of gamma inteterferon including human and rat gamma interferon; it does not, however, bind significantly to murine IFN-γ. Deleting the B8R gene prevents the impairment of IFN-γ in humans. Deletion of the B8R gene results in enhanced safety witout a concomitant reduction in immunogenicity.

### Transgene Insertions

In various embodiments, additional transgenes may be inserted into the vector. In various embodiments, one, two or three transgenes are inserted into the locus of the deleted B8R gene. In some strains, in addition to the transgene(s) present at the site of the B8R deletion, the strain also has, at least one transgene is inserted into an additional locus on the vaccinia virus that is not the locus of the deleted B8R gene. In various embodiments, at least one transgene is inserted into boundaries of the 5p deletions, at least one transgene is inserted into the boundaries of the 3p deletions or both. In various, embodiiments at least three, four, five or more transgenes are inserted into the modified vaccinia virus genome.

### Methods of treatment

### Pharmaceutical Composition, Administration, and Doses

Therapeutic compositions containing recombinant vaccinia virus vectors of the invention can be prepared using methods known in the art. For example, such compositions can be prepared using, e.g., physiologically acceptable carriers, excipients or stabilizers (Remington's Pharmaceutical Sciences 16th edition, Osol, A. Ed. (1980); incorporated herein by reference), and in a desired form, e.g., in the form of lyophilized formulations or aqueous solutions.

To induce oncolysis, kill cells, inhibit growth, inhibit metastases, decrease tumor size and otherwise reverse or reduce the malignant phenotype of tumor cells, using the methods and compositions of the present invention, one may contact a tumor with the modified vaccinia virus, e.g., by administration of the vaccinia virus to a patient having cancer by way of, for instance, one or more of the routes of administration described herein. The route of administration may vary with the location and nature of the cancer, and may include, e.g., intradermal, transdermal, parenteral, intravenous, intramuscular, intranasal, subcutaneous, regional (e.g., in the proximity of a tumor, particularly with the vasculature or adjacent vasculature of a tumor), percutaneous, intratracheal, intraperitoneal, intraarterial, intravesical, intratumoral, inhalation, perfusion, lavage, and oral administration and formulation.

The term "intravascular" is understood to refer to delivery into the vasculature of a patient, meaning into, within, or in a vessel or vessels of the patient. In certain embodiments, the administration is into a vessel considered to be a vein (intravenous), while in others administration is into a vessel considered to be an artery. Veins include, but are not limited to, the internal jugular vein, a peripheral vein, a coronary vein, a hepatic vein, the portal vein, great saphenous vein, the pulmonary vein, superior vena cava, inferior vena cava, a gastric vein, a splenic vein, inferior mesenteric vein, superior mesenteric vein, cephalic vein, and/or femoral vein. Arteries include, but are not limited to, coronary artery, pulmonary artery, brachial artery, internal carotid artery, aortic arch, femoral artery, peripheral artery, and/or ciliary artery. It is contemplated that delivery may be through or to an arteriole or capillary.

Intratumoral injection, or injection directly into the tumor vasculature is specifically contemplated for discrete, solid, accessible tumors. Local, regional or systemic administration also may be appropriate. The viral particles may advantageously be contacted by administering multiple injections to the tumor, spaced, for example, at approximately 1 cm intervals. In the case of surgical intervention, the present invention may be used preoperatively, such as to render an inoperable tumor subject to resection. Continuous administration also may be applied where appropriate, for example, by implanting a catheter into a tumor or into tumor vasculature. Such continuous perfusion may take place, for example, for a period of from about 1-2 hours, to about 2-6 hours, to about 6-12 hours, or about 12-24 hours following the initiation of treatment. Generally, the dose of the therapeutic composition via continuous perfusion may be equivalent to that given by a single or multiple injections, adjusted over a period of time during which the perfusion occurs. It is further contemplated that limb perfusion may be used to administer therapeutic compositions of the present invention, particularly in the treatment of melanomas and sarcomas.

Treatment regimens may vary, and often depend on tumor type, tumor location, disease progression, and health and age of the patient. Certain types of tumor will require more aggressive treatment, while at the same time, certain patients cannot tolerate more taxing protocols. The clinician will be best suited to make such decisions based on the known efficacy and toxicity (if any) of the therapeutic formulations. In certain embodiments, the tumor being treated may not, at least initially, be resectable. Treatments with the therapeutic agent of the disclosure may increase the resectability of the tumor due to shrinkage at the margins or by elimination of certain particularly invasive portions. Following treatments, resection may be possible. Additional treatments subsequent to resection will serve to eliminate microscopic residual disease at the tumor site.

The treatments may include various "unit doses." Unit dose is defined as containing a predetermined-quantity of the therapeutic composition. The quantity to be administered, and the particular route and formulation, are within the skill of those in the clinical arts. A unit dose need not be administered as a single injection but may comprise continuous infusion over a set period of time. Unit dose of the present invention may conveniently be described in terms of plaque forming units (pfu) for a viral construct. Unit doses may range from 10³, 10⁴, 10⁵, 10⁶, 10⁷, 10⁸, 10⁹, 10¹⁰, 10¹¹, 10¹², to 10¹³ pfu and higher. Additionally or alternatively, depending on the kind of virus and the titer attainable, one may deliver 1 to 100, 10 to 50, 100-1000, or up to about or at least about 1×10⁴, 1×10⁵, 1×10⁶, 1×10⁷, 1×10⁸, 1×10⁹, 1×10¹⁰, 1×10¹¹, 1×10¹², 1×10¹³, 1×10¹⁴, or 1×10¹⁵ or higher infectious viral particles (vp), including all values and ranges there between, to the tumor or tumor site.

Another method of delivery of the recombinant vaccinia virus genome disclosed herein to cancer or tumor cells may be via intratumoral injection. However, the pharmaceutical compositions disclosed herein may alternatively be administered parenterally, intravenously, intradermally, intramuscularly, transdermally or even intraperitoneally as described in U.S. Pat. No. 5,543,158; U.S. Pat. No. 5,641,515 and U.S. Pat. No. 5,399,363 (each specifically incorporated herein by reference in its entirety). Injection of nucleic acid constructs may be delivered by syringe or any other method used for injection of a solution, as long as the expression construct can pass through the particular gauge of needle required for injection. An exemplary needleless injection system that may be used for the administration of recombinant vaccinia viruses described herein is exemplified in U.S. Pat. No. 5,846,233. This system features a nozzle defining an ampule chamber for holding the solution and an energy device for pushing the solution out of the nozzle to the site of delivery. Another exemplary syringe system is one that permits multiple injections of predetermined quantities of a solution precisely at any depth (U.S. Pat. No. 5,846,225).

Mixtures of the viral particles or nucleic acids described herein may be prepared in water suitably mixed with one or more excipients, carriers, or diluents. Dispersions may also be prepared in glycerol, liquid polyethylene glycols, and mixtures thereof and in oils. Under ordinary conditions of storage and use, these preparations may contain a preservative to prevent the growth of microorganisms. The pharmaceutical forms suitable for injectable use include sterile aqueous solutions or dispersions and sterile powders for the extemporaneous preparation of sterile injectable solutions or dispersions (U.S. Pat. No. 5,466,468, specifically incorporated herein by reference in its entirety). In all cases the form may be sterile and may be fluid to the extent that easy syringability exists. It may be stable under the conditions of manufacture and storage and must be preserved against the contaminating action of microorganisms, such as bacteria and fungi. The carrier can be a solvent or dispersion medium containing, for example, water, ethanol, polyol (e.g., glycerol, propylene glycol, and liquid polyethylene glycol, and the like), suitable mixtures thereof, and/or vegetable oils. Proper fluidity may be maintained, for example, by the use of a coating, such as lecithin, by the maintenance of the required particle size in the case of dispersion and by the use of surfactants. The prevention of the action of microorganisms can be brought about by various antibacterial and antifungal agents, for example, parabens, chlorobutanol, phenol, sorbic acid, thimerosal, and the like. In many cases, it will be preferable to include isotonic agents, for example, sugars or sodium chloride. Prolonged absorption of the injectable compositions can be brought about by the use in the compositions of agents delaying absorption, for example, aluminum monostearate and gelatin.

For parenteral administration in an aqueous solution, for example, the solution may be suitably buffered if necessary and the liquid diluent first rendered isotonic with sufficient saline or glucose. These particular aqueous solutions are especially suitable for intravenous, intramuscular, subcutaneous, intratumoral and intraperitoneal administration. In this connection, sterile aqueous media that can be employed will be known to those of skill in the art in light of the present disclosure. For example, one dosage may be dissolved in 1 ml of isotonic NaCl solution and either added to 1000 ml of hypodermoclysis fluid or injected at the proposed site of infusion. Some variation in dosage will necessarily occur depending on the condition of the subject being treated. The person responsible for administration will, in any event, determine the appropriate dose for the individual subject. Moreover, for human administration, preparations should meet sterility, pyrogenicity, general safety, and purity standards as required by FDA Office of Biologics standards.

As used herein, "carrier" includes any and all solvents, dispersion media, vehicles, coatings, diluents, antibacterial and antifungal agents, isotonic and absorption delaying agents, buffers, carrier solutions, suspensions, colloids, and the like. The use of such media and agents for pharmaceutical active substances is well known in the art. Except insofar as any conventional media or agent is incompatible with the active ingredient, its use in the therapeutic compositions is contemplated. Supplementary active ingredients can also be incorporated into the compositions. The phrase "pharmaceutically acceptable" or "pharmacologically-acceptable" refers to molecular entities and compositions that do not produce an allergic or similar untoward reaction when administered to a human. The preparation of an aqueous composition that contains a protein as an active ingredient is well understood in the art. Typically, such compositions are prepared as injectables, either as liquid solutions or suspensions; solid forms suitable for solution in, or suspension in, liquid prior to injection can also be prepared.

### Cancer

The recombinant vaccinia virus disclosed herein can be administered to a mammalian subject, such as a human, suffering from a cell proliferation disorder, such as cancer, e.g., to kill cancer cells directly by oncolysis and/or to enhance the effectiveness of the adaptive immune response against the target cancer cells. In some embodiments, the cell proliferation disorder is a cancer, such as leukemia, lymphoma, liver cancer, bone cancer, lung cancer, brain cancer, bladder cancer, gastrointestinal cancer, breast cancer, cardiac cancer, cervical cancer, uterine cancer, head and neck cancer, gallbladder cancer, laryngeal cancer, lip and oral cavity cancer, ocular cancer, melanoma, pancreatic cancer, prostate cancer, colorectal cancer, testicular cancer, or throat cancer. In particular cases, the cell proliferation disorder may be a cancer selected from the group consisting of acute lymphoblastic leukemia (ALL), acute myeloid leukemia (AML), chronic lymphocytic leukemia (CLL), chronic myelogenous leukemia (CML), adrenocortical carcinoma, AIDS-related lymphoma, primary CNS lymphoma, anal cancer, appendix cancer, astrocytoma, atypical teratoid/rhabdoid tumor, basal cell carcinoma, bile duct cancer, extrahepatic cancer, ewing sarcoma family, osteosarcoma and malignant fibrous histiocytoma, central nervous system embryonal tumors, central nervous system germ cell tumors, craniopharyngioma, ependymoma, bronchial tumors, burkitt lymphoma, carcinoid tumor, primary lymphoma, chordoma, chronic myeloproliferative neoplasms, colon cancer, extrahepatic bile duct cancer, ductal carcinoma in situ (DCIS), endometrial cancer, ependymoma, esophageal cancer, esthesioneuroblastoma, extracranial germ cell tumor, extragonadal germ cell tumor, fallopian tube cancer, fibrous histiocytoma of bone, gastrointestinal carcinoid tumor, gastrointestinal stromal tumors (GIST), testicular germ cell tumor, gestational trophoblastic disease, glioma, childhood brain stem glioma, hairy cell leukemia, hepatocellular cancer, langerhans cell histiocytosis, hodgkin lymphoma, hypopharyngeal cancer, islet cell tumors, pancreatic neuroendocrine tumors, wilms tumor and other childhood kidney tumors, langerhans cell histiocytosis, small cell lung cancer, cutaneous T-cell lymphoma, intraocular melanoma, merkel cell carcinoma, mesothelioma, metastatic squamous neck cancer, midline tract carcinoma, multiple endocrine neoplasia syndromes, multiple myeloma/plasma cell neoplasm, myelodysplastic syndromes, nasal cavity and paranasal sinus cancer, nasopharyngeal cancer, neuroblastoma, non-hodgkin lymphoma (NHL), non-small cell lung cancer (NSCLC), epithelial ovarian cancer, germ cell ovarian cancer, low malignant potential ovarian cancer, pancreatic neuroendocrine tumors, papillomatosis, paraganglioma, paranasal sinus and nasal cavity cancer, parathyroid cancer, penile cancer, pharyngeal cancer, pheochromocytoma, pituitary tumor, pleuropulmonary blastoma, primary peritoneal cancer, rectal cancer, retinoblastoma, rhabdomyosarcoma, salivary gland cancer, kaposi sarcoma, rhabdomyosarcoma, sezary syndrome, small intestine cancer, soft tissue sarcoma, throat cancer, thymoma and thymic carcinoma, thyroid cancer, transitional cell cancer of the renal pelvis and ureter, urethral cancer, endometrial uterine cancer, uterine sarcoma, vaginal cancer, vulvar cancer, and Waldenström macroglobulinemia.

A physician having ordinary skill in the art can readily determine an effective amount of the recombinant vaccinia virus vector for administration to a mammalian subject (e.g., a human) in need thereof. For example, a physician may start prescribing doses of recombinant vaccinia virus vector at levels lower than that required in order to achieve the desired therapeutic effect and gradually increase the dosage until the desired effect is achieved. Alternatively, a physician may begin a treatment regimen by administering a dose of recombinant vaccinia virus vector and subsequently administer progressively lower doses until a therapeutic effect is achieved (e.g., a reduction in the volume of one or more tumors). In general, a suitable daily dose of a recombinant vaccinia virus vector of the invention will be an amount of the recombinant vaccinia virus vector which is the lowest dose effective to produce a therapeutic effect. A daily dose of a therapeutic composition of the recombinant vaccinia virus vector of the invention may be administered as a single dose or as two, three, four, five, six or more doses administered separately at appropriate intervals throughout the day, week, month, or year, optionally, in unit dosage forms. While it is possible for the recombinant vaccinia virus vector of the invention to be administered alone, it may also be administered as a pharmaceutical formulation in combination with excipients, carriers, and optionally, additional therapeutic agents.

Recombinant vaccinia virus vectors of the invention can be monitored for their ability to attenuate the progression of a cell proliferation disease, such as cancer, by any of a variety of methods known in the art. For instance, a physician may monitor the response of a mammalian subject (e.g., a human) to treatment with recombinant vaccinia virus vector of the invention by analyzing the volume of one or more tumors in the patient. Alternatively, a physician may monitor the responsiveness of a subject (e.g., a human) t to treatment with recombinant vaccinia virus vector of the invention by analyzing the T-reg cell population in the lymph of a particular subject. For instance, a physician may withdraw a sample from a mammalian subject (e.g., a human) and determine the quantity or density of cancer cells using established procedures, such as fluorescence activated cell sorting. A finding that the quantity of cancer cells in the sample has decreased (e.g., by 1%, 2%, 3%, 4%, 5%, 6%, 7%, 8%, 9%, 10%, 20%, 30%, 40%, 50%, 60%, 70%, 80%, 90%, 95%, 96%, 97%, 98%, 99%, or more) relative to the quantity of cancer cells in a sample obtained from the subject prior to administration of the recombinant vaccinia virus may be an indication that the vaccinia virus administration is effectively treating the cancer.

### Assays for Measuring Virus Characteristics

Assays known in the art to measure the tumor spreading and virulence of a virus include but are not limited to measuring plaque size, syncytia formation, and/or comet assays (EEVs). Assays known in the art to measure the immunostimulatory activity of a virus include but are not limited to NK activation (measured in % CD69 expression), NK degranulation (measured in fold increase of CD107a), and/or T-cell priming assays. Assays known in the art to measure the selectivity of a virus include, but are not limited to, tail pox lesions, biodistribution, and/or body mass measurements.

### Examples of Proteins Encoded by Vaccinia Virus Genes

As used below, the term "location" refers to the location of the gene with respect to the deleted nucleic acids in exemplary vaccinia virus vectors described herein. For various genes, amino acid sequence information and protein accession ID numbers are provided.

**Table 2. Examples of proteins encoded by Copenhagen Vaccinia genes deleted in CopMD5p vector**

| **SEQ ID NO.** | **Gene** | **Protein Accession ID** | **Amino Acid Sequence** | **Locatio n** |
|---|---|---|---|---|
| SEQ ID NO:23 | C2L (26% 5') | AAA47999. 1 | MESVIFSINGEIIQVNKEIITASPYNFFKRIQD HHLKDEAIILNGINYHAFESLLDYIRWKKIN ITINNVEMILVAAIIIDVPPVVDLCVKTMIH NINSTNCIRMFNFSKRYGIKKLYNASMSEII NNITAVTSDPEFGKLSKDELTTILSHENVNV NHEDVTAMILLKWIHKNPNDVDIINILHPK FMTNTMRNAISLLGLTISKSTKPVTRNGIK HNIVVIKNSDYISTITHYSPRTEYWTIVGNT DRQFYNANVLHNCLYIIGGMINNRHVYSV SRVDLETKKWKTVTNMSSLKSEVSTCVND GKLYVIGGLEFSISTGVAEYLKHGTSKWIR LPNLITPRYSGASVFVNDDIYVMGGVYTTY EKYVVLNDVECFTKNRWIKKSPMPRHHSI VYAVEYDGDIYVITGITHETRNYLYKYIVK EDKWIELYMYFNHVGKMFVCSCGDYILIIA DAKYEYYPKSNTWNLFDMSTRNIEYYDMF TKDETPKCNVTHKSLPSFLSNCEKQFLQ | Inside Deletion |
| SEQ ID NO:24 | C1L | AAA48000. 1 | MVKNNKISNSCRMIMSTNPNNILMRHLKN LTDDEFKCIIHRSSDFLYLSDSDYTSITKETL VSEIVEEYPDDCNKILAIIFLVLDKDIDVDIE TKLKPKPAVRFAILDKMTEDIKLTDLVRHY FRYIEQDIPLGPLFKKIDSYRTRAINKYSKE LGLATEYFNKYGHLMFYTLPIPYNRFFCRN SIGFLAVLSPTIGHVKAFYKFIEYVSIDDRR KFKKELMSK | Inside Deletion |
| SEQ ID NO:25 | N1L | AAA48001. 1 | MRTLLIRYILWRNDNDQTYYNDDFKKLML LDELVDDGDVCTLIKNMRMTLSDGPLLDR LNQPVNNIEDAKRMIAISAKVARDIGERSEI RWEESFTILFRMIETYFDDLMIDLYGEK | Inside Deletion |
| SEQ ID NO:26 | N2L | AAA48002. 1 | MTSSAMDNNEPKVLEMVYDATILPEGSSM DPNIMDCINRHINMCIQRTYSSSIIAILNRFL TMNKDELNNTQCHIIKEFMTYEQMAIDHY GEYVNAILYQIRKRPNQHHTIDLFKKIKRTP YDTFKVDPVEFVKKVIGFVSILNKYKPVYS YVLYENVLYDEFKCFINYVETKYF | Inside Deletion |
| SEQ ID NO:27 | M1L | AAA48003. 1 | MIFVIESKLLQIYRNRNRNINFYTTMDNIMS AEYYLSLYAKYNSKNLDVFRNMLQAIEPS GNNYHILHAYCGIKGLDERFVEELLHRGYS PNETDDDGNYPLHIASKINNNRIVAMLLTH GADPNACDKHNKTPLYYLSGTDDEVIERIN LLVQYGAKINNSVDEEGCGPLLACTDPSER VFKKIMSIGFEARIVDKFGKNHIHRHLMSD NPKASTISWMMKLGISPSKPDHDGNTPLHI VCSKTVKNVDIIDLLLPSTDVNKQNKFGDS PLTLLIKTLSPAHLINKLLSTSNVITDQTVNI CIFYDRDDVLEIINDKGKQYDSTDFKMAVE VGSIRCVKYLLDNDIICEDAMYYAVLSEYE TMVDYLLFNHFSVDSVVNGHTCMSECVRL NNPVILSKLMLHNPTSETMYLTMKAIEKD KLDKSIIIPFIAYFVLMHPDFCKNRRYFTSY KRFVTDYVHEGVSYEVFDDYF | Inside Deletion |
| SEQ ID NO:28 | M2L | AAA48004. 1 | MVYKLVLLFCIASLGYSVEYKNTICPPRQD YRYWYFAAELTIGVNYDINSTIIGECHMSE SYIDRNANIVLTGYGLEINMTIMDTDQRFV AAAEGVGKDNKLSVLLFTTQRLDKVHHNI SVTITCMEMNCGTTKYDSDLPESIHKSSSC DITINGSCVTCVNLETDPTKINPHYLHPKDK YLYHNSEYGMRGSYGVTFIDELNQCLLDIK ELSYDICYRE | Inside Deletion |
| SEQ ID NO:29 | HR/K1 L | AAA48005. 1 | MDLSRINTWKSKQLKSFLSSKDTFKADVH GHSALYYAIADNNVRLVCTLLNAGALKNL LENEFPLHQAATLEDTKIVKILLFSGMDDS QFDDKGNTALYYAVDSGNMQTVKLFVKK NWRLMFYGKTGWKTSFYHAVMLNDVSIV SYFLSEIPSTFDLAILLSCIHTTIKNGHVDM MILLLDYMTSTNTNNSLLFIPDIKLAIDNKD IEMLQALFKYDINIYSVNLENVLLDDAEITK MIIEKHVEYKSDSYTKDLDIVKNNKLDEIIS KNKELRLMYVNCVKKN | Inside Deletion |
| SEQ ID NO:30 | SPI-3/K2L | AAA48006. 1 | MIALLILSLTCSVSTYRLQGFTNAGIVAYK NIQDDNIVFSPFGYSFSMFMSLLPASGNTRI ELLKTMDLRKRDLGPAFTELISGLAKLKTS KYTYTDLTYQSFVDNTVCIKPLYYQQYHR FGLYRLNFRRDAVNKINSIVERRSGMSNVV DSNMLDNNTLWAIINTIYFKGTWQYPFDIT KTRNASFTNKYGTKTVPMMNVVTKLQGN TITIDDEEYDMVRLPYKDANISMYLAIGDN MTHFTDSITAAKLDYWSFQLGNKVYNLKL PKFSIENKRDIKSIAEMMAPSMFNPDNASF KHMTRDPLYIYKMFQNAKIDVDEQGTVAE | Inside Deletion |
| | | | ASTIMVATARSSPEKLEFNTPFVFIIRHDITG FILFMGKVESP | |
| SEQ ID NO:31 | K ORF A | AAA48007. 1 | MGHIITYCQVHTNISILIRKAHHIIFFVIDCD CISLQFSNYVHHGNRFRTVLISKTSIACFSDI KRILPCTFKIYSINDCP | Inside Deletion |
| SEQ ID NO:32 | K ORF B | AAA48008. 1 | MGTVFVPYLLVKLALRVLVISNGYCHVPL KYIVLMIAHRVLLSSILESTTLDIPDLRSTIE LILLTASRLKFNLYRPNL | Inside Deletion |
| SEQ ID NO:33 | K3L | AAA48009. 1 | MLAFCYSLPNAGDVIKGRVYEKDYALYIY LFDYPHSEAILAESVKMHMDRYVEYRDKL VGKTVKVKVIRVDYTKGYIDVNYKRMCR HQ | Inside Deletion |
| SEQ ID NO:34 | K4L | AAA48010. 1 | MNPDNTIAVITETIPIGMQFDKVYLSTFNM WREILSNTTKTLDISSFYWSLSDEVGTNFG TIILNEIVQLPKRGVRVRVAVNKSNKPLKD VERLQMAGVEVRYIDITNILGGVLHTKFWI SDNTHIYLGSANMDWRSLTQVKELGIAIFN NRNLAADLTQIFEVYWYLGVNNLPYNWK NFYPSYYNTDHPLSINVSGVPHSVFIASAPQ QLCTMERTNDLTALLSCIRNASKFVYVSV MNFIPIIYSKAGKILFWPYIEDELRRSAIDRQ VSVKLLISCWQRSSFIMRNFLRSIAMLKSK NIDIEVKLFIVPDADPPIPYSRVNHAKYMVT DKTAYIGTSNWTGNYFTDTCGASINITPDD GLGLRQQLEDIFMRDWNSKYSYELYDTSP TKRCKLLKNMKQCTNDIYCDEIQPEKEIPE YSLE | Inside Deletion |
| SEQ ID NO:35 | K5L | AAA48011. 1 | MGATISILASYDNPNLFTAMILMSPLVNAD AVSRLNLLAAKLMGTITPNAPVGKLCPESV SRDMDKVYKYQYDPLINHEKIKAGFASQV LKATNKVRKIISKINTPRLSYSREQTMRLV MFQVHIISCNMQIVIE | Inside Deletion |
| SEQ ID NO:36 | K6L | AAA48012. 1 | MSANCMFNLDNDYIYWKPITYPKALVFISH GAGKHSGRYDELAENISSLGILVFSHDHIG HGRSNGEKMMIDDFGTARGNY | Inside Deletion |
| SEQ ID NO:37 | K7R | AAA48013. 1 | MATKLDYEDAVFYFVDDDKICSRDSIIDLI DEYITWRNHVIVFNKDITSCGRLYKELMKF DDVAIRYYGIDKINEIVEAMSEGDHYINFT KVHDQESLFATIGICAKITEHWGYKKISESR FQSLGNITDLMTDDNINILILFLEKKLN | Inside Deletion |
| SEQ ID NO:38 | F1L | AAA48014. 1 | MLSMFMCNNIVDYVDDIDNGIVQDIEDEA SNNVDHDYVYPLPENMVYRFDKSTNILDY LSTERDHVMMAVRYYMSKQRLDDLYRQL PTKTRSYIDIINIYCDKVSNDYNRDMNIMY DMASTKSFTVYDINNEVNTILMDNKGLGV RLATISFITELGRRCMNPVKTIKMFTLLSHT ICDDCFVDYITDISPPDNTIPNTSTREYLKLI GITAIMFATYKTLKYMIG | Inside Deletion |
| SEQ ID NO:39 | DUT/F 2L | AAA48015. 1 | MFNMNINSPVRFVKETNRAKSPTRQSPGA AGYDLYSAYDYTIPPGERQLIKTDISMSMP KICYGRIAPRSGLSLKGIDIGGGVIDEDYRG NIGVILINNGKCTFNVNTGDRIAQLIYQRIY YPELEEVQSLDSTNRGDQGFGSTGLR | Inside Deletion |
| SEQ ID NO:40 | F3L (75% 3') | AAA48016. 1 | MPIFVNTVYCKNILALSMTKKFKTIIDAIGG NIIVNSTILKKLSPYFRTHLRQKYTKNKDPV TRVCLDLDIHSLTSIVIYSYTGKVYIDSHNV VNLLRASILTSVEFIIYTCINFILRDFRKEYC VECYMMGIEYGLSNLLCHTKNFIAKHFLEL EDDIIDNFDYLSMKLILESDELNVPDEDYV VDFVIKWYIKRRNKLGNLLLLIKNVIRSNY LSPRGINNVKWILDCTKIFHCDKQPRKSYK YPFIEYPMNMDQIIDIFHMCTSTHVGEVVY LIGGWMNNEIHNNAIAVNYISNNWIPIPPM NSPRLYATGIPANNKLYVVGGLPNPTSVER WFHGDAAWVNMPSLLKPRCNPAVASINN VIYVMGGHSETDTTTEYLLPNHDQWQFGP STYYPHYKSCALVFGRRLFLVGRNAEFYC ESSNTWTLIDDPIYPRDNPELIIVDNKLLLIG GFYRGSYIDTIEVYNHHTYSWNIWDGK | Inside Deletion |

**Table 3. Examples of proteins encoded by Copenhagen Vaccinia genes deleted in CopMD3p vector**

| **SEQ ID NO** | **Gene** | **Protein Accession ID** | **Amino Acid Sequence** | **Locatio n** |
|---|---|---|---|---|
| SEQ ID NO:10 8B14R (41% 3') | | AAA48211. 1 | MNHCLLAISAVYFKAKWLTPFEKEFTSDYP FYVSPTEMVDVSMMSMYGELFNHASVKE SFGNFSIIELPYVGDTSMMVILPDKIDGLESI EQNLTDTNFKKWCNSLDAMFIDVHIPKFK VTGSYNLVDTLVKSGLTEVFGSTGDYSNM CNLDVSVDAMIHKTYIDVNEEYTEAAAAT CALVSDCASTITNEFCVDHPFIYVIRHVDG KILFVGRYCSPTTNC | Inside Deletion |
| SEQ ID NO:10 9B15R | | AAA48212. 1 | MTANFSTHVFSPQHCGCDRLTSIDDVKQCL TEYIYWSSYAYRNRQCAGQLYSTLLSFRD DAELVFIDIRELVKNMPWDDVKDCTEIIRC YIPDEQKTIREISAIIGLCAYAATYWGGEDH PTSNSLNALFVMLEMLNYVDYNIIFRRMN | Inside Deletion |
| SEQ ID NO:11 0B ORF E | | AAA48213. 1 | MYNSSIHTPEYDVIIHVIEHLKHHKQCVQT VTSGMVFTSPVSSSICTKSDDGRNLSDGFL LIRYITTDDFCTIFDIIPRHIFYQLANVDEH | Inside Deletion |
| SEQ ID NO:11 1B16R | | AAA48214. 1 | MSILPVIFLPIFFYSSFVQTFNASECIDKGXY FASFMELENEPVILPCPQINTLSSGYNILDIL WEKRGADNDRIIPIDNGSNMLILNPTQSDS GIYICITTNETYCDMMSLNLTIVSVSESNIDF ISYPQIVNERSTGEMVCPNINAFIASNVNAD IIWSGHRRLRNKRLKQRTPGIITIEDVRKND AGYYTCVLEYIYGGKTYNVTRIVKLEVRD KIIHPTMQLPEGVVTSIGSNLTIACRVSLRPP TTDADVFWISNGMYYEEDDGDGDGRISVA NKIYMTDKRRVITSRLNINPVKEEDATTFT CMAFTIPSISKTVTVSIT | Inside Deletion |
| SEQ ID NO:11 2B ORF F | | AAA48215. 1 | MVIIPGVRCLSLLFLRRRCPLHIISAFTLLAI NALILGHTISPVDLSFTICGYEIKSIFDSETDT IVKFNDIMSQ | Inside Deletion |
| SEQ ID NO:11 3 | B17L | AAA48216. 1 | MSRKFMQVYEYDREQYLDEFIEDRYNDSFI TSPEYYSAEKYMCRYTTLNHNCVNVRRCA LDSKLLHDIITNCKIYNNIELVRATKFVYYL DLIKCNWVSKVGDSVLYPVIFITHTSTRNL DKVSVKTYKGVKVKKLNRCADHAIVINPF VKFKLTLPNKTSHAKVLVTFCKLRTDITPV EAPLPGNVLVYTFPDINKRIPGYIHVNIEGCI DGMIYINSSKFACVLKLHRSMYRIPPFPIDI | Inside Deletion |
| | | | CSCCSQYTNDDIEIPIHDLIKDVAIFKNKET VYYLKLNNKTIARFTYFNNIDTAITQEHEY VKIALGIVCKLMINNMHSIVGVNHSNTFVN CLLEDNV | |
| SEQ ID NO:11 4B18R | | AAA48217. 1 | MSRRLIYVLNINRKSTHKIQENEIYTYFSHC NIDHTSTELDFVVKNYDLNRRQHVTGYTA LHCYLYNNYFTNDVLKILLNHDVNVTMKT SSGRMPVYILLTRCCNISHDVVIDMIDKDK NHLSHRDYSNLLLEYIKSRYMLLKEEDIDE NIVSTLLDKGIDPNFKQDGYTALHYYYLCL AHVYKPGECRKPITIKKAKRIISLFIQHGAN LNALDNCGNTPFHLYLSIEMCNNIHMTKM LLTFNPNFKICNNHGLTPILCYITSDYIQHDI LVMLIHHYETNVGEMPIDERRMIVFEFIKT YSTRPADSITYLMNRFKNINIYTRYEGKTLL HVACEYNNTQVIDYLIRINGDINALTDNNK HATQLIIDNKENSPYTINCLLYILRYIVDKN VIRSLVDQLPSLPIFDIKSFEKFISYCILLDDT FYDRHVKNRDSKTYRYAFSKYMSFDKYD GIITKCHDETMLLKLSTVLDTTLYAVLRCH NSRKLRRYLTELKKYNNDKSFKIYSNIMNE RYLNVYYKDMYVSKVYDKLFPVFTDKNC LLTLLPSEIIYEILYMLTINDLYNISYPPTKV | Inside Deletion |
| SEQ ID NO:11 5B19R | | AAA48218. 1 | MTMKMMVHIYFVSLSLLLLLFHSYAIDIEN EITEFFNKMRDTLPAKDSKWLNPACMFGG TMNDMATLGEPFSAKCPPIEDSLLSHRYKD YVVKWERLEKNRRRQVSNKRVKHGDLWI ANYTSKFSNRRYLCTVTTKNGDCVQGIVR SHIKKPPSCIPKTYELGTHDKYGIDLYCGIL YAKHYNNITWYKDNKEINIDDIKYSQTGK ELIIHNPELEDSGRYDCYVHYDDVRIKNDI VVSRCKILTVIPSQDHRFKLILDPKINVTIGE PANITCTAVSTSLLIDDVLIEWENPSGWLIG FDFDVYSVLTSRGGITEATLYFENVTEEYIG NTYKCRGHNYYFEKTLTTTVVLE | Inside Deletion |
| SEQ ID NO:11 6B20R | | AAA48219. 1 | MDEDTRLSRYLYLTDREHINVDSIKQLCKI SDPNACYRCGCTALHEYFYNYRSVNGKYK YRYNGYYQYYSSSDYENYNEYYYDDYDR TGMNSESDSESDNISIKTEYENEYEFYDETQ DQSTQHNDL | Inside Deletion |
| SEQ ID NO:11 7B21R | | AAA48220. 1 | MSLESFIITTFNNNSSTNIDNMCHLYVKVCP SSLLFRLFVECCDINKLVEGTTPLHCYLMN EGFESSVLKNLLKEYVMNTFNVHDIHYTNI | Inside Deletion |
| SEQ ID NO:11 8B22R | | AAA48221. 1 | MISLSFLIHNPLKKWKLKPSISINGYRSTFT MAFPCAQFRPCHCHATKDSLNTVADVRHC LTEYILWVSHRWTHRESAGSLYRLLISFRT DATELFGGELKDSLPWDNIDNCVEIIKCFIR NDSMKTAEELRAIIGLCTQSAIVSGRVFND KYIDILLMLRKILNENDYLTLLDHIRTAKY | Inside Deletion |
| SEQ ID NO:11 9B23R | | AAA48222. 1 | MIAFIIFREIGIISTRIAMDYCGRECTILCRLL DEDVTYKKIKLEIETCHNLSKHIDRRGNNA LHCYVSNKCDTDIKIVRLLLSRGVERLCRN NEGLTPLGAYSKHRYVKSQIVHLLISSYSN SSNELKSNINDFDLSSDNIDLRLLKYLIVDK RIRPSKNTNYAINGLGLVDIYVTTPNPRPEV LLWLLKSECYSTGYVFRTCMYNSDMCKNS LHYYISSHRESQSLSKDVIKCLINNNVSIHG RDEGGSLPIQYYWSFSTIDIEIVKLLLIKDV DTCRVYDVSPILEAYYLNKRFRVTPYNVD MEIVNLLIERRHTLVDVMRSITSYDSREYN HYIIDNILKRFRQQDESIVQAMLINYLHYG DMVVRCMLDNGQQLSSARLLC | Inside Deletion |
| SEQ ID NO:12 0B24R | | AAA48223. 1 | MYGLILSRFNNCGYHCYETILIDVFDILSKY MDDIDMIDNENKTLLYYAVDVNNIQFAKR LLEYGASVTTSRSIINTAIQKSSYQRENKTRI VDLLLSYHPTLETMIDAFNRDIRYLYPEPLF ACIRYALILDDDFPSKVSMISPVIIRN | Inside Deletion |
| SEQ ID NO:12 1B ORF G | | AAA48224. 1 | MRRCIHIKERKIHMTNIVDRNVTFILTVVH KYVRYVPHTVANDAHNLVHLAHLIHFIIYF FIIRDVRKKKKKKKKKNRTIYFFSNVYARHI K | Inside Deletion |
| SEQ ID NO:12 2B25R | | AAA48225. 1 | MSRINITKKIYCSVFLFLFLFLSYISNYEKVN DEMYEMGEMDEIVSIVRDSMWYIPNVFMD DGKNEGHVSVNNVCHMYFTFFDVDTSSHL FKLVIKHCDLNKRGNSPLHCYTMNTRFNPS VLKILLHHGMRNFDSKDEKGHHYLIHSLSI DNKIFDILTDTIDDFSKSSDLLLCYLRYKFN GSLNYYVLYKGSDPNCADEDELTSLHYYC KHISTFYKSNYYKLSHTKMRAEKRFIYAIID YGANINAVTHLPSTVYQT | Inside Deletion |
| SEQ ID NO:12 3B26R | | AAA48226. 1 | MEQTLTRLHTYLQQYTKHSPRVVYALLSR GYVIILIVHPSWNDCATGHILIMLLNWHEQ KEEGQHLLYLFIKHNQGYTLNILRYLLDRF DIQKDEYIYRLSKL | Inside Deletion |
| SEQ ID NO:12 4B27R | | AAA48227. 1 | MLPHTSDTTSTFRLKTVFDLVFENRNIIYKA DVVNDIIHHRLKVSLPMIKSLFYKMSEFSP | Inside Deletion |
| | | | YDDYYVKKILAYCLLRDESFAELHSKFCLN EDYKSVFMKNISFDKIDSIIVT | |
| SEQ ID NO:12 5B28R | | AAA48228. 1 | MKSVLYSYILFLSCIIINGRDIAPHAPSDGKC KDNEYKRHNLCPGTYASRLCDSKTNTQCT PCGSGTFTSRNNHLPACLSCNGRRDRVTLL TIESVNALPDIIVFSKDHPDARHVFPKQNVE | Inside Deletion |
| SEQ ID NO:12 6 | C23L/B 29R (44% 5') | AAA48229. 1 | MHVPASLQQSSSSSSCTEEENKHHMGIDV IIKVTKQDQTPTNDKICQSVTEITESESDPDP EVESEDDSTSVEDVDPPTTYYSIIGGGLRM NFGFTKCPQIKSISESADGNTVNARLSSVSP GQGKDSPAITREEALAMIKDCEVSIDIRCSE EEKDSDIKTHPVLGSNISHKKVSYEDIIGSTI VDTKCVKNLEFSVRIGDMCKESSELEVKD GFKYVDGSASEGATDDTSLIDSTKLKACV | Inside Deletion |

### Examples

The following examples are put forth so as to provide those of ordinary skill in the art with a description of how the compositions and methods claimed herein are performed, made, and evaluated, and are intended to be purely exemplary of the invention and are not intended to limit the scope of what the inventor regards as her invention.

### Example 1 - Creation of the CopMD5p3p "SKV-B8R+" Recombinant Vaccinia virus

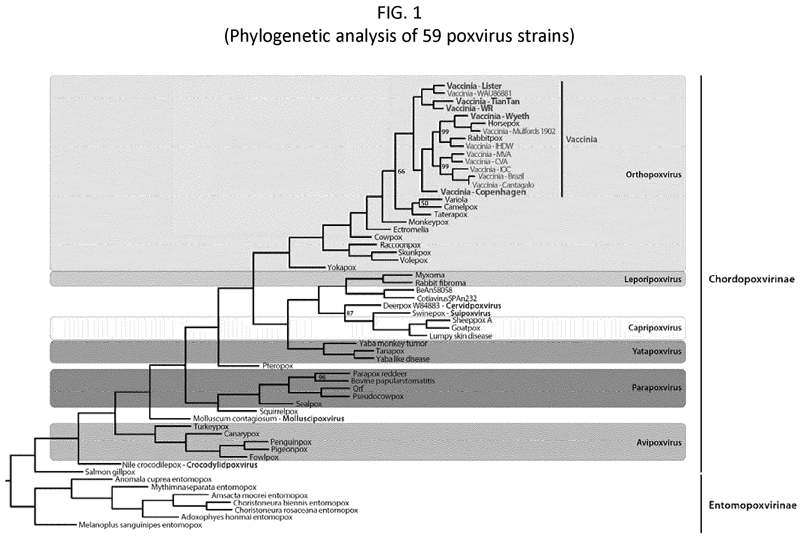

The open reading frames (ORFs) from 59 poxvirus strains were clustered into orthologs and aligned at the amino acid level (see FIG. 1 for phylogenetic analysis). Bayesian analysis was performed to determine relatedness of all strains. Poxviruses are very diverse in gene content and host range. There are several naturally occurring Vaccinia wild-type strains, which are different from one another.

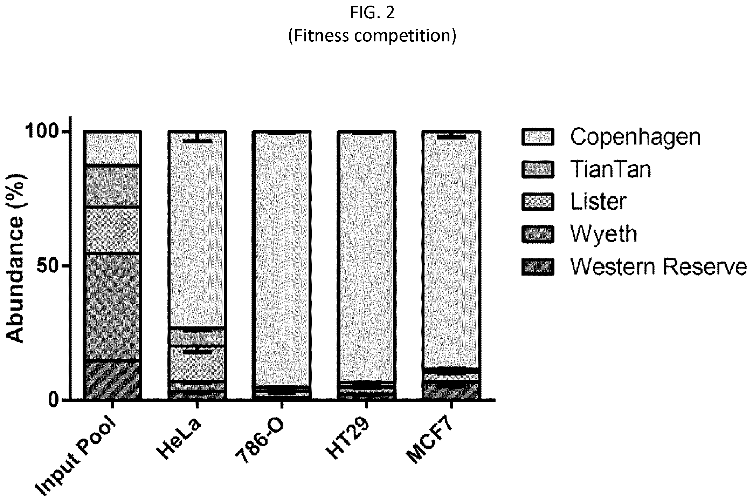

Five Vaccinia wild type strains (Copenhagen, TianTan, Lister, Wyeth, and Western Reserve) were mixed at equal plaque forming unit counts and sequenced with NGS (Input pool). The resulting mixture was passaged three times in different cancer cell lines (HeLa, 786-O, HT29, MCF7). The final population was sequenced with NGS illumina sequencing. Reads (short DNA fragments) were assigned to various strains based on sequence identity and used to calculate the percent of each strain in the final population. The relative abundance of the different viral strains was then quantified. As shown in FIG. 2, the Copenhagen strain was the most abundant vaccinia strain after three passages in any of the four cancer cell lines indicating that this strain was able to outgrow other strains and therefore replicates faster.

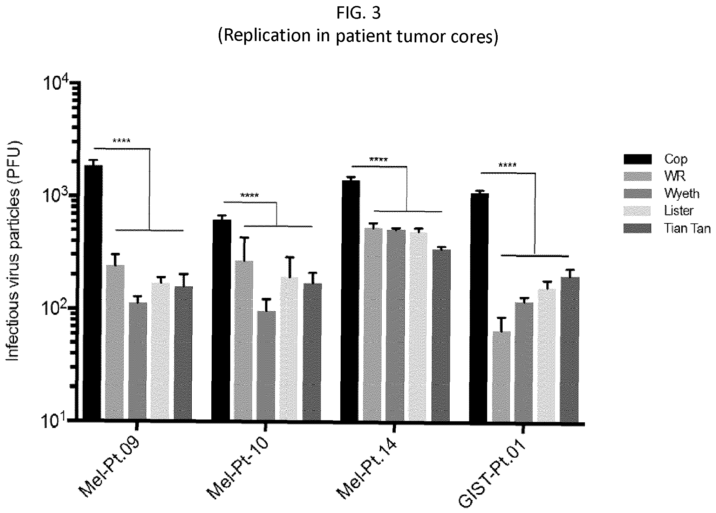

Different Vaccinia wild type strains were also used to infect at low PFU (1 × 10⁴) various patient tumor cores. Each strain infected on average 4 replicates each containing three 2 × 2 mm tumor cores. Replication was assessed through virus titering and is expressed as plaque forming units (PFU) as shown in FIG. 3. The Copenhagen strain grows to higher titers than other strains and therefore replicates faster in patient ex-vivo samples. Patient ex-vivo cores are a good mimic of a patient's 3D tumor.

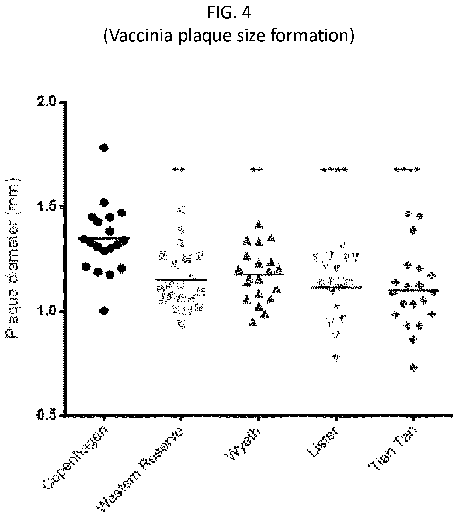

Vaccinia wild-type strains were then subjected to a plaque assay on U2-OS cells with a 3% CMC overlay. Two days past infection, 20-30 plaques for each strain were measured for their size. Plaque size measurements for Copenhagen, Western Reserve, Wyeth, Lister, and Tian Tan are shown in FIG. 4. Plaque formation is affected by the ability of the virus to replicate, spread, and kill. The larger plaque sizes observed for the Copenhagen strain suggest that this strain is superior in these abilities, which are important for the development of an oncolytic virus.

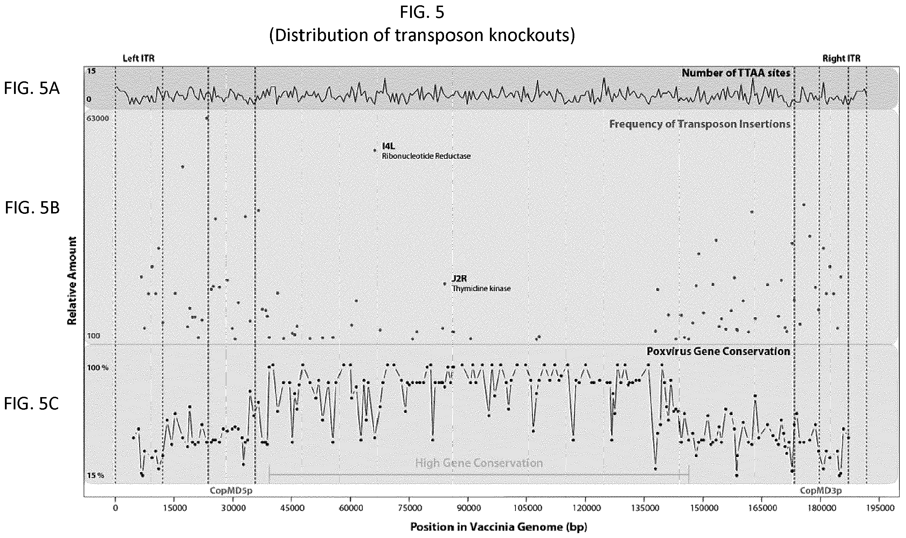

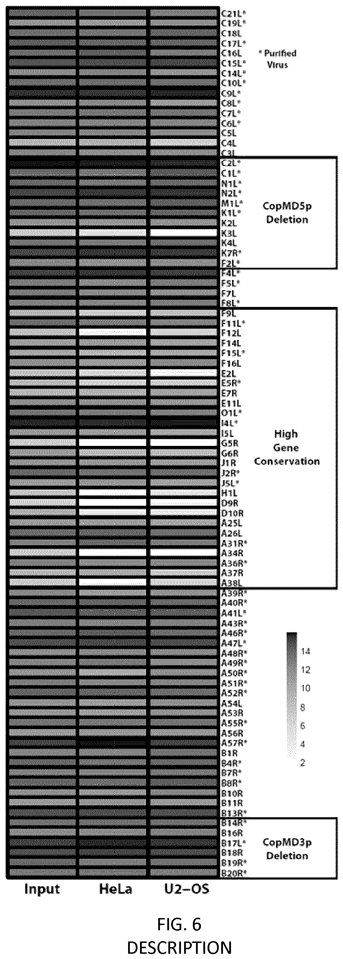

Then, the number of TTAA sites across 1kb regions in Vaccinia Copenhagen genome were counted (see FIG. 5A). Ilumina NGS sequencing was combined and used to identify Transposon Insertion Sites (Tn-Seq). The input library was passaged three times in either HeLa or U2-OS cells after which frequencies of transposon knockouts were determined. The frequency of a knockout directly corresponds to the amount of reads supporting the event (see FIG. 5B and FIG. 6).

Finally, all 59 poxvirus genomes from FIG. 1 were used to find ORFs and clustered into orthologous groups. Groups containing Copenhagen genes were plotted based on location of the gene in the Copenhagen genome (x-axis) and size of the group (y-axis). When all 59 species share the same gene the conservation is considered to be 100%. The TTAA motif is required for a transposon insertion and this motif is ubiquitous along the genome, meaning transposons can insert anywhere in the genome. However, it was noted that transposons insert preferentially in areas of low poxvirus gene conservation. While sequencing transposon knockouts, major deletions were identified and labelled as CopMD5p and CopMD3p (see FIG. 5C and FIG. 6). Genes that are present in the middle of the genome and that have an elevated gene conservation (FIG. 5C) are important for viral replication. This is because knocking these genes out with transposon insertions causes a decrease in fitness (less frequency after passaging). Genes that are part of the major deletions CopMD5p and CopMD3p were found to be less important for viral replication as their deletion does not impact fitness.

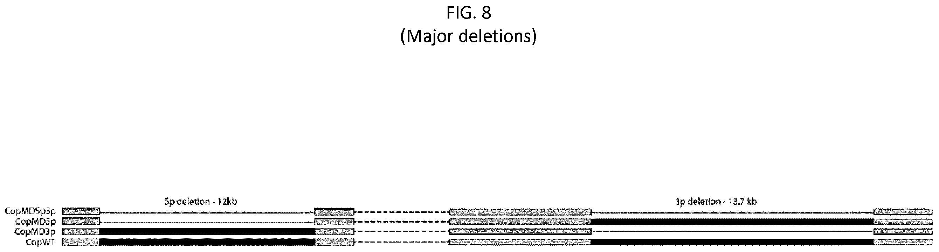

Illumina NGS deep sequencing revealed presence of major deletions during the plaque purification process. CopMD5p and CopMD3p represent clones, which were plaque purified and found to harbor major genomic deletions. These 2 clones were used to co-infect a monolayer of HeLa cells at a high MOI (MOI 10) to induce recombination. Random plaque picking and PCR revealed presence of a double deleted CopMD5p3p which contained both genome deletions (see FIG. 8). These 2 deletions were combined and purified to give a replicating virus, referred to herein as "CopMD5p3p", that exhibits deletions in the C2L, C1L, N1L, N2L, MIL, M2L, K1L, K2L, K3L, K4L, K5L, K6L, K7R, F1L, F2L, F3L, B14R, B15R, B16R, B17L, B18R, B19R, and B20R genes, as well as single deletions in each of the ITR genes B21R, B22R, B23R, B24R, B25R, B26R, B27R, B28R, and B29R. As used herein, "CopWT" refers to wild-type Copenhagen vaccinia virus, "CopMD5p" refers to a Copenhagen vaccinia virus harboring deletions in representative 5' genes (C2L, C1L, N1L, N2L, MIL, M2L, K1L, K2L, K3L, K4L, K5L, K6L, K7R, F1L, F2L, F3L), and "CopMD3p" refers to a Copenhagen vaccinia virus harboring deletions in representative 3' genes (B14R, B15R, B16R, B17L, B18R, B19R, and B20R) as well as single deletions in each of the ITR genes B21R, B22R, B23R, B24R, B25R, B26R, B27R, B28R, and B29R.

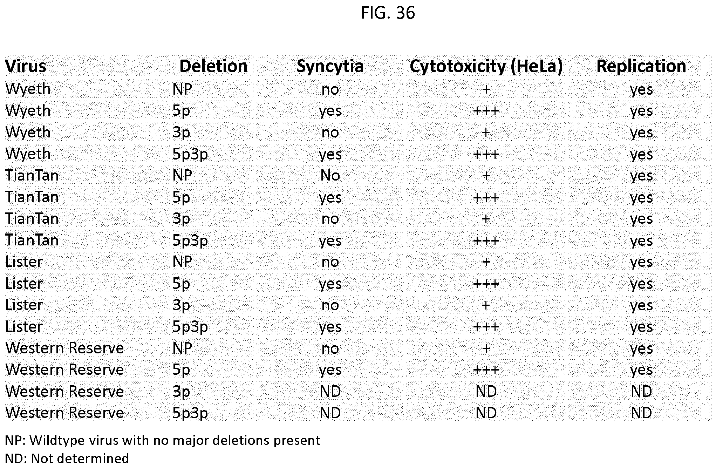

The 59 poxvirus geneomes were then assessed for the presence of these 31 genes deleted in the CopMD5p3p. Homology searches were used to query poxviruses from other clades with amino acid seuqences of Table 2 genes from the Copenhagen genome. As shown in FIG 36, the percentage of these 32 genes present in various poxvirus strains decreases with increasing divergence from the Copenhagen strain (each dot on the plot represents one poxvirus genome). However, a majority of the members of the vaccinia family, comprise at least 85% of the the genes which are deleted in the CopMD5p3p recombinant vector.

### Example 2 - Cancer cell death

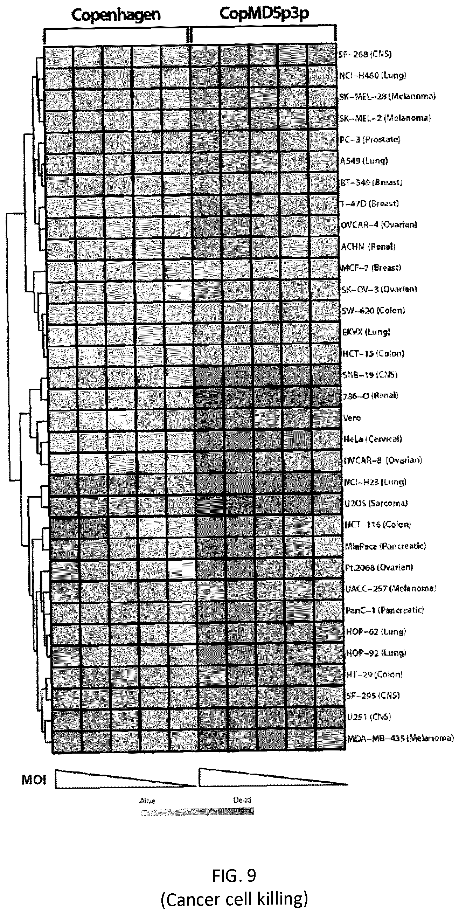

Cancer cells were infected with CopMD5p3p at a range of MOIs (1 to 0.01) in 24-well plates in 4 replicates. Two days post infection with virus, plates were stained with crystal violet. Crystal violet stain was dissolved into SDS and read by spectrophotometry. Data is represented as percent of non-infected cells (see FIG. 9). This data shows that the majority of cancer cell lines die faster when exposed to the CopMD5p3p virus.

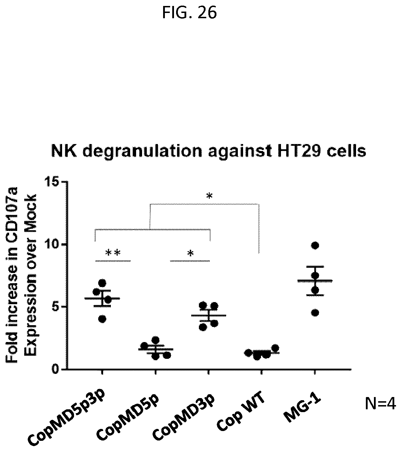

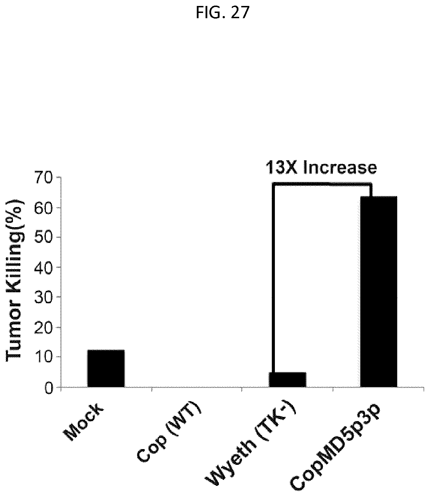

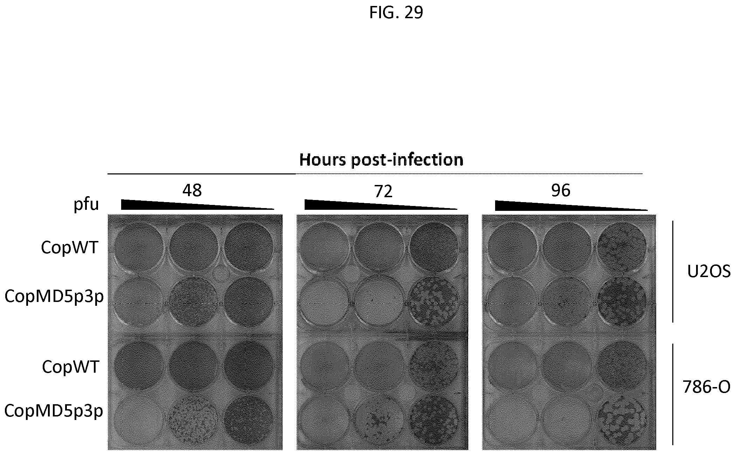

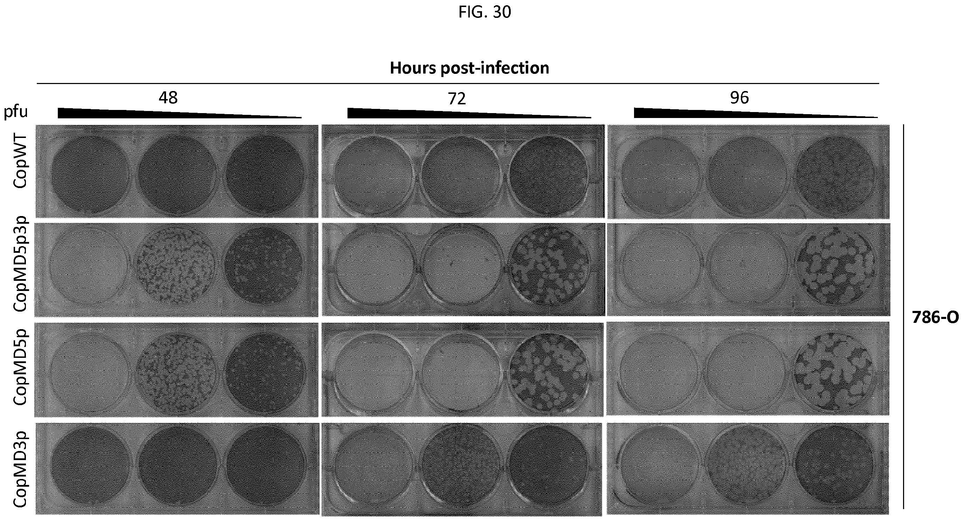

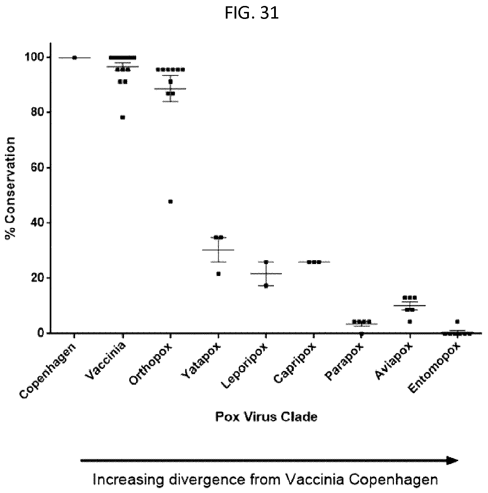

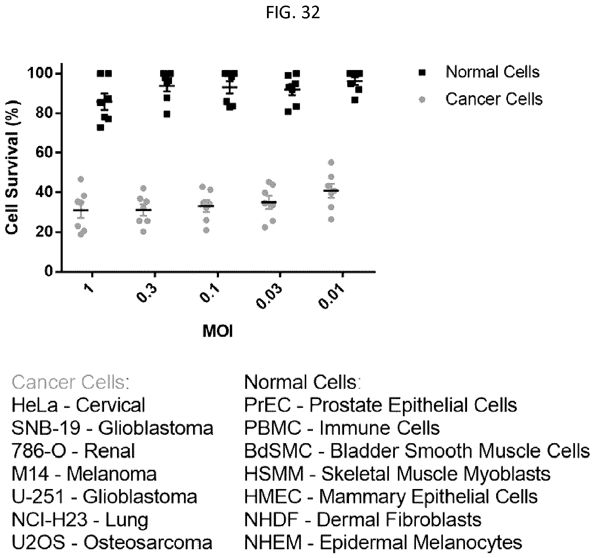

The ability of wild-type Copenhagen vaccinia virus and several modified Copenhagen vaccinia virions to induce an anti-tumor immune response and to propagate in various cancer cell lines is also shown in FIGS. 26, 27, and 29-35.

### Example 3 - Growth in cancer cells

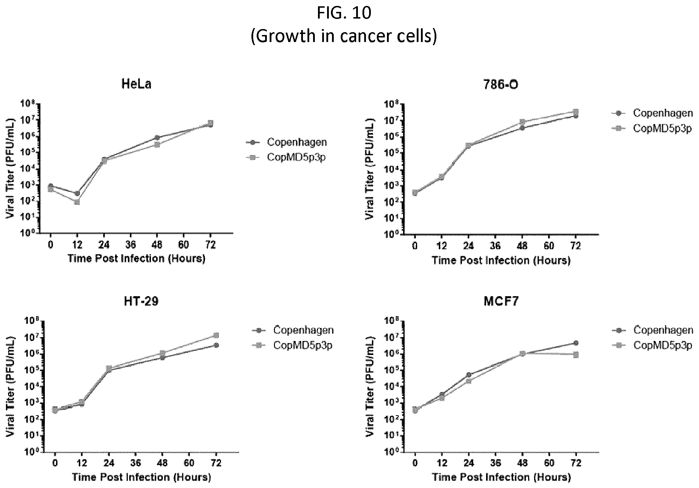

Four cancer cell lines were infected with CopMD5p3p at a low MOI (0.001) in 24-well plates in triplicates, and at different time points, the virus was collected and tittered. Time 0h represents input. The growth curves of HeLa, 786-O, HT-29, and MCF7 are shown in FIG. 10. This data shows that the modified CopMD5p3p virus is not impaired in its ability to grow in vitro. This means that the virus is replication competent, even in presence of interferon response. The ability to replicate in mammalian cell lines provides another important advantage. As such, viruses may be manufactured with enhanced speed and efficiency.

### Example 4 - Growth in patient tumor samples

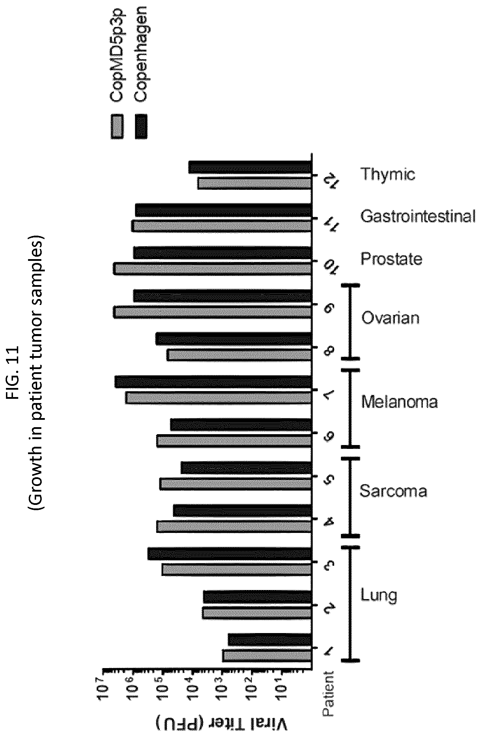

Patient tumor samples were obtained immediately after surgery and cut into 2mm × 2mm cores. Three cores were infected with a small amount of virus (1 × 10⁴ PFU), either wild-type Copenhagen or CopMD5p3p. After 72h virus output was assessed by plaque assay and final Viral Titer expressed as PFU (see FIG. 11). This data shows that the modified CopMD5p3p virus can replicate in fresh patient tumor samples. Replication in patient tumor samples is a good model of replication in a patient 3D tumor.

### Example 5 - Syncytia in U2-OS cells

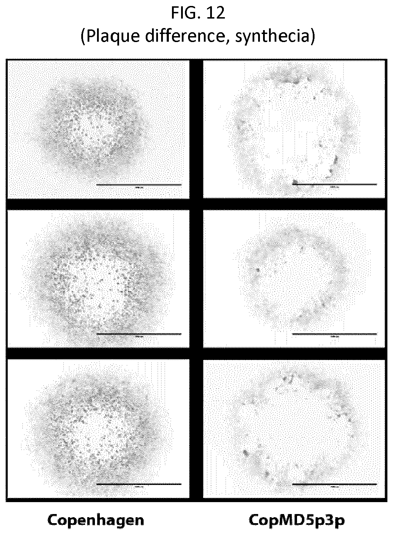

Monolayers of U2-OS cells were infected with either Copenhagen wild-type or CopMD5p3p virus. After 2h, the media was changed for overlay media as done for a plaque assay. At 48h post infection, pictures were taken with EVOS to assess plaque phenotype (see FIG. 12). Cell fusion, also known as syncytia, is thought to help the virus spread, since uninfected cells merge with infected cells. Additionally, it has been shown that fused cells are immunogenic and in the case of cancer cells can help initiate an anti-tumor immune response. See, e.g., http://cancerres.aacrjournals.org/content/62/22/6566.long.

### Example 6 - Syncytia in 786-O cells

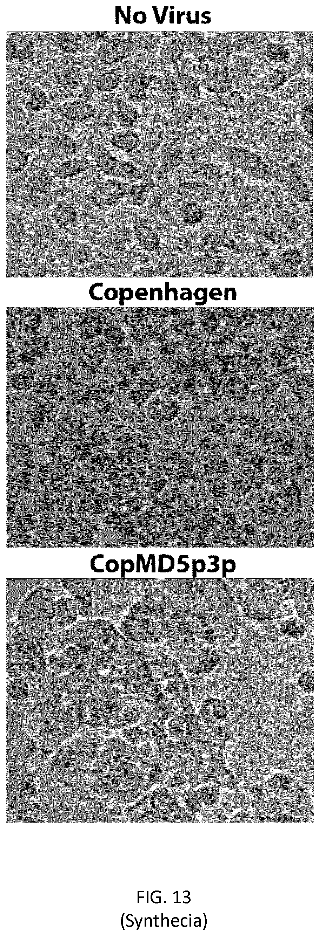

Monolayers of 786-O cells were infected with either Copenhagen wild-type or CopMD5p3p virus. After 24h pictures were taken with EVOS at 10X magnification (see FIG. 13). This is additional evidence for the occurrence of syncytia. In FIG. 12, the phenotype of a plaque is shown. In the current experiment, monolayers of cells were infected without overlay. Most cells infected by the CopMD5p3p virus have fused.

### Example 7 - Tumor control and weight loss in mouse model

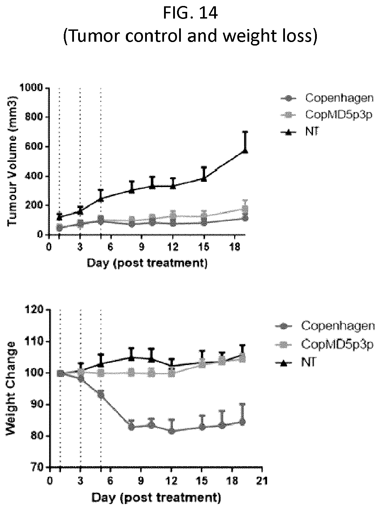

Nude CD-1 (Crl:CD1-Foxn1nu) mice were seeded with HT-29 human colon cancer xenograft (5e6 cells). Once subcutaneous tumours have established an approximate 5mm × 5mm size, mice were treated three times (dashed lines) 24h apart with 1 × 10⁷ PFU of either vaccinia virus intravenously. Mice were measured approximately every other day for tumor size and weight loss (see FIG. 14). This experiment shows that CopMD5p3p is a much safer virus because it does not cause any weight loss or other signs of sickness in immunocompromised nude mice. This experiment also shows CopMD5p3p is able to control tumor growth similarly to the parental Copenhagen wild-type virus.

### Example 8 - Pox lesion formation

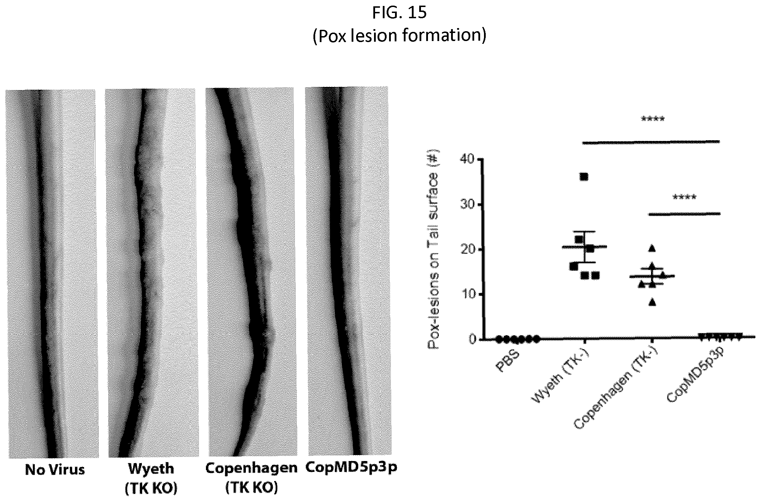

Nude CD-1 mice were treated once with 1 × 10⁷ PFU of either vaccinia virus intravenously, six mice per group. Two weeks post treatment, mice were sacrificed and pictures of tails were taken. Pox lesions on tails were counted manually on every mouse tail. Representative pictures shown in FIG. 15. This experiment shows that CopMD5p3p is a much safer virus because it does not cause any pox lesions in immunocompromised nude mice. This is important since prior Oncolytic Vaccinia clinical data has shown patients developing pox lesions upon treatment. Knockout of thymidine kinase (TK) is a popular way of increasing the safety of an OV (oncolytic virus), currently present in a Phase III Oncolytic Vaccinia and in FDA approved Oncolytic T-Vec. The data shows that deleting TK does not play a crucial role in this assay, where mice develop pox lesions when challenged with TK deleted viruses, but do not develop pox lesions with CopMD5p3p which has an intact TK.

### Example 9 - IVIS Bio-distribution of Vaccinia after systemic administration

Vaccinia viruses wild-type Wyeth, wild-type Copenhagen, and CopMD5p3p were engineered to express Firefly Luciferase (Fluc) and YFP through transfection of infected cells with a pSEM1 plasmid replacing TK with Fluc and YFP. Viruses were plaque purified and expanded. All viruses are TK knockouts and encode functional Fluc in their TK locus.

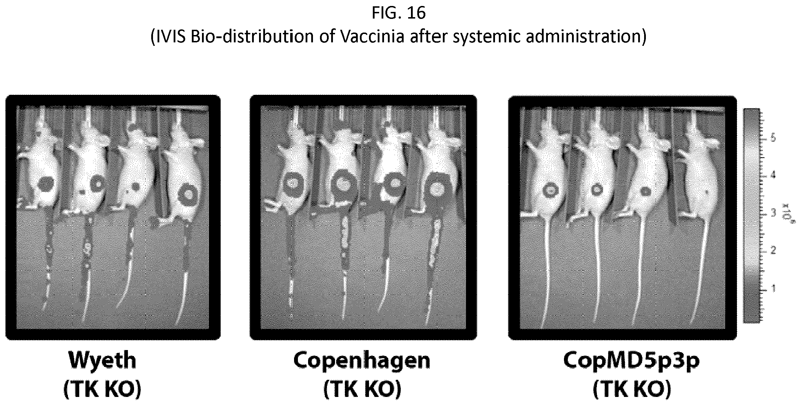

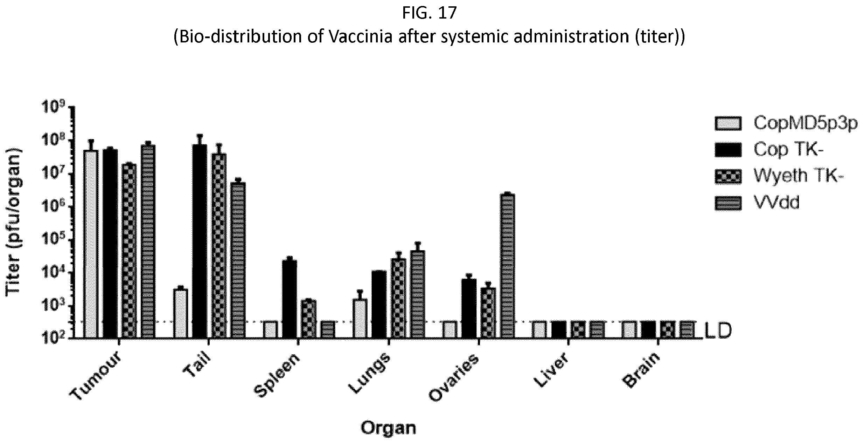

Nude CD-1 mice were then seeded with HT-29 human colon cancer xenograft. Once subcutaneous tumors have established an approximate 5mm × 5mm size, mice were treated once with 1e7 PFU of either vaccinia Fluc encoding virus intravenously, four mice per group. Four days post treatment, mice were injected i.p. (intraperitoneal) with luciferin and imaged with IVIS for presence of virus (see FIG. 16). This experiment shows that CopMD5p3p is a much safer virus because it is more specific to the tumor. Other viruses show off target replication in the tail, muscle, paws and intra-nasal cavity. CopMD5p3p is only localized in the tumor. As shown in previous FIGS. 15 and 16, there is less detectable CopMD5p3p in the tail compared to the other strains. FIG. 17 shows that CopMD5p3p also has lower titers in other organs when compared to other oncolytic Vaccinia. Since the CopMD5p3p replicates at the same level as the other viruses in the tumor but less in off-target tissues, CopMD5p3p fits the profile of an oncolytic virus better.

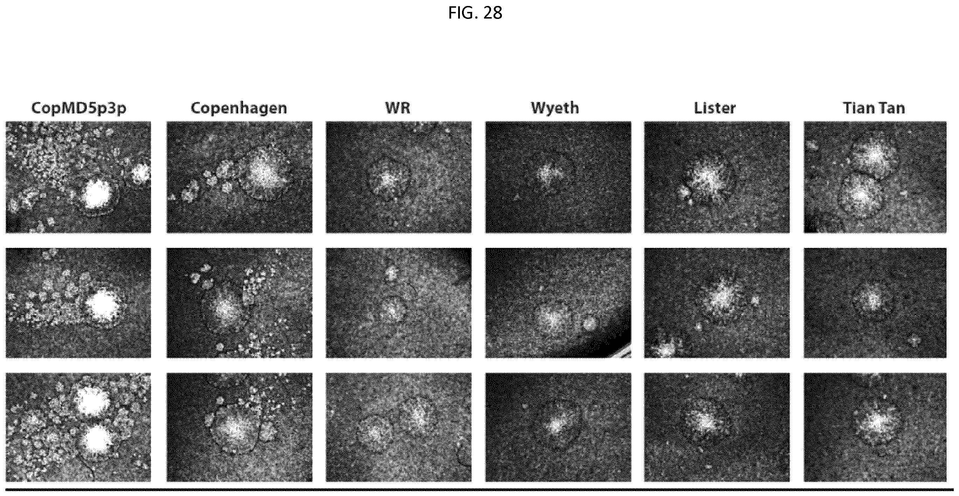

An additional example of the biodistribution of various vaccinia viral vectors, including the wild-type Copenhagen vaccinia virus and several modified Copenhagen vaccinia virions is shown in FIG. 28.

### Example 10 - Immunogenicity of Vaccinia in human PBMCs

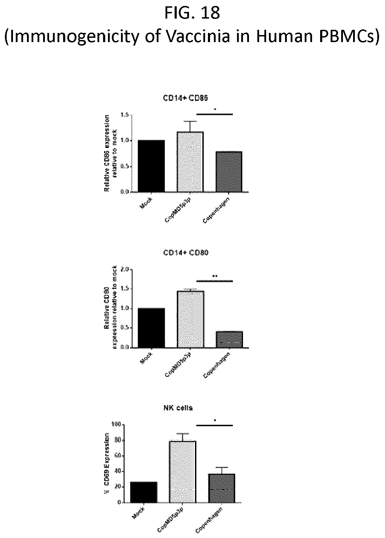

PBMCs were isolated from blood of healthy human donors (n = 2). PBMCs were incubated with either Vaccinia for 24h and checked for early activation markers using Flow Cytometry (see FIG. 18). This experiment shows that CopMD5p3p is more immunogenic and more readily detectable by immune cells. We believe that this is a desirable trait, since OVs replicating in tumor tissue need to activate immune cells for a successful anti-tumor immune response.

### Example 11 - Immunogenicity of Vaccinia in mouse splenocytes

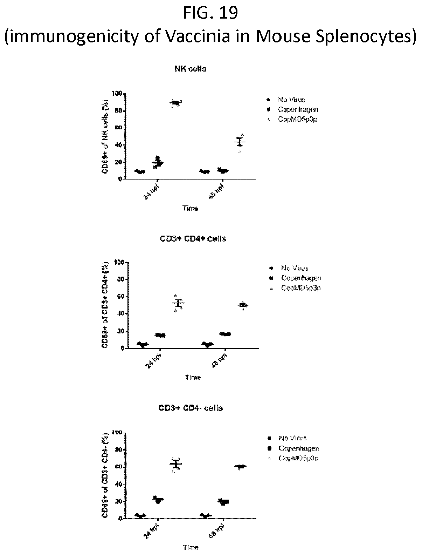

Immune competent Balb/C mice were injected with 1 × 10⁷ Vaccinia PFU Vaccinia virus intravenously. After one or two days, mice were sacrificed, spleens were harvested and analyzed for immune activation using Flow Cytometry (see FIG. 19). This experiment shows that CopMD5p3p is more immunogenic and more readily detectable by mouse immune cells. This data complements nicely the previous FIG. 18, since most of the in vivo experiments are done in mice.

### Example 12 - Immunogenicity of Vaccinia in human cells

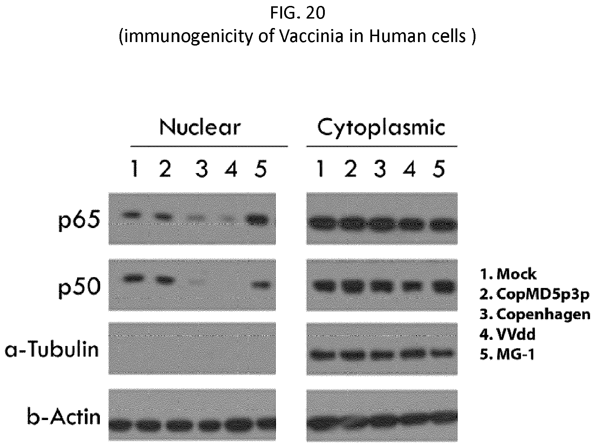

Human cancer cells 786-O were infected at an MOI of 0.01 with either virus. The next day, cells were harvested and nuclei and cytoplasm were separated by cell fractionation. Protein was extracted from each fraction and blotted for NF-kB subunits p65 and p50 (see FIG. 20). NF-kB immune transcription factor initiated an immune response once it's subunit p65 and p50 are translocated to the nucleus. Some viruses are immunosuppressive and block this translocation, preventing an immune response. Suppressing NF-kB function is counterintuitive to the goal of using oncolytic viruses in combination with immunotherapeutic approaches. Thus, CopMD5p3p is a more advantageous virus as it behaves similarly to MG-1.

### Example 13 - Administration for the treatment of a subject

Using the methods described herein, a clinician of skill in the art can administer to a subject (e.g., a patient) a pharmaceutical composition containing a recombinant vaccinia virus vector described herein to treat cancer or tumor cells. The cancer may be, for example, leukemia, lymphoma, liver cancer, bone cancer, lung cancer, brain cancer, bladder cancer, gastrointestinal cancer, breast cancer, cardiac cancer, cervical cancer, uterine cancer, head and neck cancer, gallbladder cancer, laryngeal cancer, lip and oral cavity cancer, ocular cancer, melanoma, pancreatic cancer, prostate cancer, colorectal cancer, testicular cancer, or throat cancer, among others.

For instance, a clinician of skill in the art may assess that a patient is suffering from cancer or tumors and may administer to the patient a therapeutically effective amount (e.g., an amount sufficient to decrease the size of the tumor) of a pharmaceutical composition containing the recombinant vaccinia virus vector disclosed herein. The pharmaceutical composition may be administered to the subject in one or more doses (e.g., 1, 2, 3, 4, 5, 6, 7, 8, 9, 10, 15, 20, 25, or more) per a specified time interval (e.g., weekly, daily, or hourly). The patient may be evaluated between doses to monitor the effectiveness of the therapy and to increase or decrease the dosage based on the patient's response. The pharmaceutical composition may be administered to the patient orally, parenterally (e.g., topically), intravenously, intramuscularly, subcutaneously, or intranasally. The treatment may involve a single dosing of the pharmaceutical composition. The treatment may involve continued dosing of the pharmaceutical composition (e.g., days, weeks, months, or years).

### Example 14 - Targeted deletions of CopMD5p and CopMd3p

The following protocol for producing modified vaccinia viral vectors utilizes techniques described, e.g., in Rintoul et al. PLoS One. 6(9): e24643 (2011), the disclosure of which is incorporated herein by reference.

Briefly, CopMD5p (Copenhagen vaccinia virus harboring deletions in 5' genes: C2L, C1L, N1L, N2L, MIL, M2L, K1L, K2L, K3L, K4L, K5L, K6L, K7R, F1L, F2L, F3L) and CopMd3p (Copenhagen vaccinia virus harboring deletions in 3' genes: (B14R, B15R, B16R, B17L, B18R, B19R, and B20R as well as single deletions in each of the ITR genes B21R, B22R, B23R, B24R, B25R, B26R, B27R, B28R, and B29R targeting recombinant constructs were synthesized by g-Block technology (IDT, Coralville Iowa). U2OS cells were infected with wildtype vaccinia virus (Wyeth, Western Reserve, Tian Tan, Lister) at an MOI of 0.01 in serum free DMEM for 1.5 hours. Viral supernatant was aspirated and U2OS cells were transfected with PCR amplified CopMD5p or CopMd3p targeting g-Blocks by Lipofectamine 2000 (Invitrogen) in OptiMEM (Gibco). DMEM supplemented with 10% FBS was added to cells 30 minutes after transfection and left overnight. The following day, transfection media was aspirated and fresh DMEM 10% FBS media was added to cells. 48 hours after infection transfection, U2OS cells were harvested and lysed by a single freeze thaw cycle. Serially diluted lysates were plated onto a confluent monolayer of U2OS cells and eGFP positive (CopMD5p targeted) or mCherry positive (CopMd3p targeted) plaques were isolated and purified through 5 rounds of plaque purifications.

Double major deleted vaccinia viruses were generated by co-infection of CopMD5p and CopMd3p deleted vaccinia viruses at an MOI of 5 for each virus in U2OS cells. Cells were harvested the next day and lysed by one round of freeze thaw. Lysates were serially diluted and plated onto a confluent monolayer of U2OS cells and selected for double positive plaques (eGFP + mCherry). Plaques were purified by 5 rounds of plaque purification.

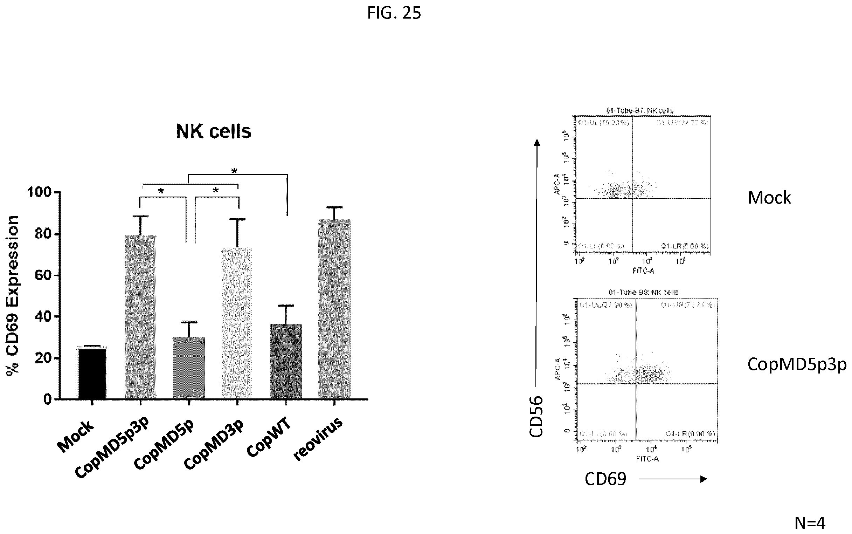

An exemplary scheme for the production of modified vaccinia virus vectors (e.g., modified vaccinia viral vectors, such as modified Copenhagen vaccinia viral vectors) of the disclosure is shown in FIG. 25.

### Example 15 - SKV-GFP (CopMD5p3p-B8R-) has similar efficacy in tumour control compared to SKV-(CopMD5p3p-B8R+)

The vaccinia virus (VV) B8R gene encodes a secreted protein with homology to gamma interferon receptor (IFN-γ). In vitro, the B8R protein binds to and neutralizes the antiviral activity of several species of gamma inteterferon including human and rat gamma interferon; it does not, however, bind significantly to murine IFN-γ. Here we describe the construction and characterization of recombinant VVs lacking the B8R gene. Homologous recombination between the targeting construct and the B8R locus resulted in the replacement of 75% of the B8R gene with the eGFP transgenes flanked by two loxP sites (SKV-GFP).

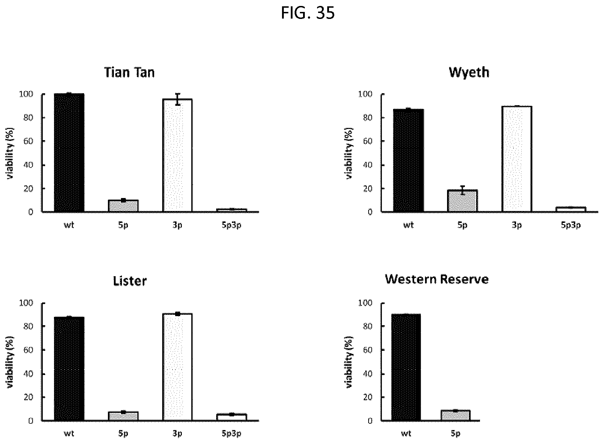

B8R- viruses showed similar efficacy to B8R+ viruses. Fig. 35. Survival of mice treated with either SKV or SKV-GFP was assessed. 5 × 10⁶ CT26-LacZ cells were seeded subcutaneously on day 0. On day 14, 16 and 18 tumours were treated at a dose of 107 pfu with an intratumoural injection of either SKV or SKV-GFP. No significant decrease in efficacy was seen whenthe viruses injected had a deletion of the B8R locus.

### Example 16 - Infection of normal versus cancer cell lines of SKV (CopMD5p3p-B8R+) virus

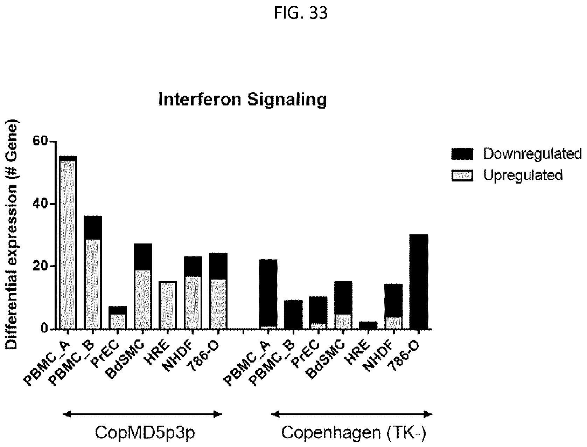

Primary health cell viability was compared to that of cancer cells. Confluent normal or cancer cells were infected at a range of MOI (pfu/cell) for 48 hrs, after which viability was quantified. As indicated in Fig 33, SKV(CopMD5p3p-B8R+) virus preferentially infects cancer cells.

### Example 17 - SKV(CopMD5p3p-B8R+)does not impair interferon signaling.

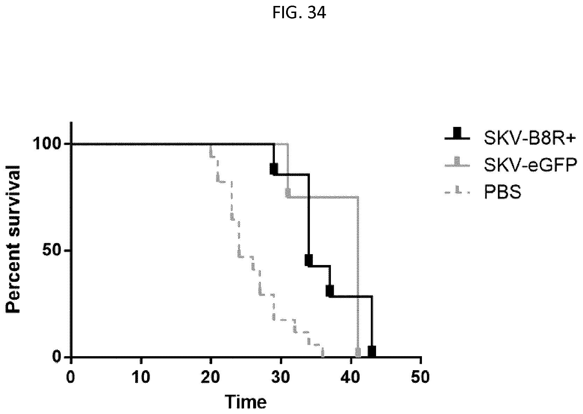

Interferon signaling was assessed by determining the number of genes in the interferon pathway that are upregulated (induced expression) or downregulated (repressed expression) in a variety of normal cell lines and one cancer cell line (786-O). FIG. 34 Confluent monolayers of 1 million cells were infected at an MOI of 3 (3e6 PFU) for 18h with either SKV-B8R+ (CopMD5p3p) or the parental Copenhagen virus strain having the TK gene disabled. RNA was sequenced using RNA-seq and gene expression of interferon genes was determined after read mapping a expression normalization. While the SKV-B8R+ (CopMD5p3p) virus mostly induces genes in the interferon pathway the parental Copenhagen represses genes. This suggests SKV-B8R+ (CopMD5p3p) is able to induce Type I Interferon signaling which is critical in viral clearance of normal cells.

### Example 18 - Major double deletions in engineered in various vaccinia strains enhance cancer cell killing in vitro

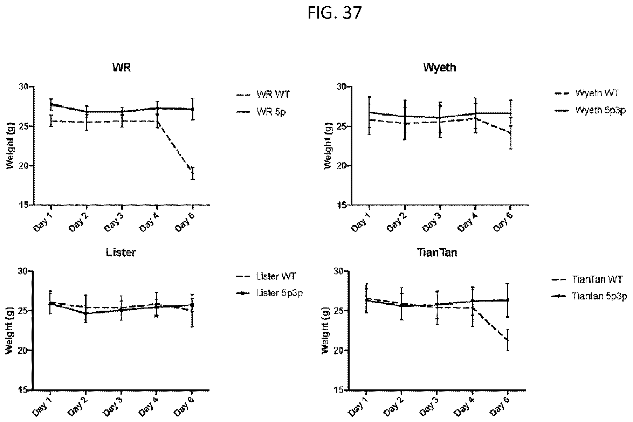

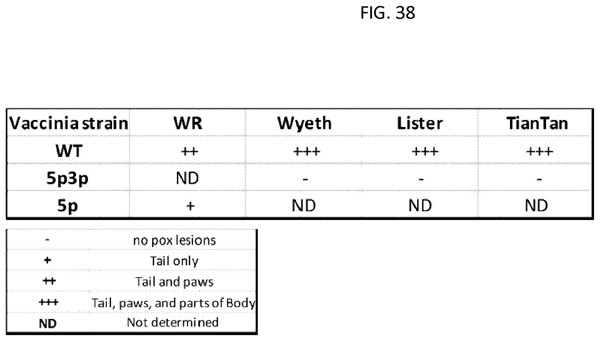

Hela cells were infected at an MOI of 0.1 with the following strains of enginnered vaccinia viruses: (1) parental wildtype virus (wt); (2) 5 prime major deleted (5p), (3) 3 prime major deleted (3p), and (4) recombined 5 prime and 3 prime major double deleted (5p3p). Cell viability was quantified by alamar blue assay 72 hours post infection. Both 5p and 5p3p major double deleted vaccinia strains are more cytotoxic in HelLa cells when compared to their parental wildtype and 3p major deleted strains. See FIG. 36. FIG. 37 depicts a summary of the major deleted Vaccinia strains, and the effect of 5p, 3p and 5p3p deletions on syncytia, cytotoxicity and replication. CD-1 nude mice were treated with 1 × 10⁷ pfu via intravenously tail vein injection and measured at the indicated timepoints. 5p3p vaccinia strains did not induce weight loss compared to wildtype strains. FIG. 38. Mice were also examined for pox lesions 6 days post-injection. 5p3p vaccinia strains do not induce pox lesions compared to wildtype strains. FIG. 39.

### Some embodiments

All publications, patents, and patent applications mentioned in this specification are incorporated herein by reference to the same extent as if each independent publication or patent application was specifically and individually indicated to be incorporated by reference.

While the invention has been described in connection with specific embodiments thereof, it will be understood that it is capable of further modifications and this application is intended to cover any variations, uses, or adaptations of the invention following, in general, the principles of the invention and including such departures from the invention that come within known or customary practice within the art to which the invention pertains and may be applied to the essential features hereinbefore set forth, and follows in the scope of the claims.

Some embodiments are within the claims.

Also disclosed herein are *inter alia* the following items (said items are not claims):
1. A nucleic acid comprising a recombinant vaccinia virus genome, said genome derived from the Vaccinia Copenhagen strain genome, wherein said recombinant vaccinia virus genome comprises a deletion of at least six vaccinia genes, one vaccinia gene from each of the following (a)-(f):
   a) F1L;
   b) N1L and B14R;
   c) M2L, K1L, and K7R;
   d) C2L, N2L, MIL, K2L, K3L, F3L, B16R, and B19R;
   e) K4L, K5L, K6L, and F2L; and
   f) B15R, B17L, B18R, and B20R and
   wherein the TK and HA genes of said genome are not deleted.
2. The nucleic acid of item 1, wherein said deletion comprise a deletions of F1L.
3. The nucleic acid of item 1, wherein said deletions comprise a deletion of N1L.
4. The nucleic acid of item 1, wherein said deletions comprise a deletion of B14R.
5. The nucleic acid of item 1, wherein said deletions comprise a deletion of M2L.
6. The nucleic acid of item 1, wherein said deletions comprise a deletion of K1L.
7. The nucleic acid of item 1, wherein said deletions comprise a deletion of K7R.
8. The nucleic acid of item 1, wherein said deletions comprise a deletion of C2L.
9. The nucleic acid of item 1, wherein said deletions comprise a deletion of N2L.
10. The nucleic acid of item 1, wherein said deletions comprise a deletion of M1L.
11. The nucleic acid of item 1, wherein said deletions comprise a deletion of K2L.
12. The nucleic acid of item 1, wherein said deletions comprise a deletion of K3L.
13. The nucleic acid of item 1, wherein said deletions comprise a deletion of F3L.
14. The nucleic acid of item 1, wherein said deletions comprise a deletion of B16R.
15. The nucleic acid of item 1, wherein said deletions comprise a deletion of B19R.
16. The nucleic acid of item 1, wherein said deletions comprise a deletion of K4L.
17. The nucleic acid of item 1, wherein said deletions comprise a deletion of K5L.
18. The nucleic acid of item 1, wherein said deletions comprise a deletion of K6L.
19. The nucleic acid of item 1, wherein said deletions comprise a deletion of F2L.
20. The nucleic acid of item 1, wherein said deletions comprise a deletion of B15R.
21. The nucleic acid of item 1, wherein said deletions comprise a deletion of B17L.
22. The nucleic acid of item 1, wherein said deletions comprise a deletion of B18R.
23. The nucleic acid of item 1, wherein said deletions comprise a deletion of B20R.
24. The nucleic acid of item 1, wherein said deletions comprise a deletion of each of said C2L, C1L, N1L, N2L, MIL, M2L, K1L, K2L, K3L, K4L, K5L, K6L, K7R, F1L, F2L, F3L, B14R, B15R, B16R, B17L, B18R, B19R, and B20R genes.
25. The nucleic acid of any one of items 1-24, wherein said recombinant vaccinia virus genome comprises a deletion of at least 1 gene selected from the group consisting of B14R, B16R, B17L, B18R, B19R, and B20R.
26. The nucleic acid of any one of items 1-25, wherein said recombinant vaccinia virus genome comprises a deletion of at least 1 gene selected from the group consisting of C2L, C1L, N1L, N2L, MIL, M2L, K1L, K2L, K3L, K4L, K5L, K6L, K7R, F1L, F2L, and F3L.
27. The nucleic acid of any one of items 1-26, wherein each of said deletions is a deletion of the entire gene encoding the corresponding polynucleotide.
28. The nucleic acid of any one of items 1-27, wherein each of said deletions is a deletion of a portion of the gene, and wherein said deletion is sufficient to render said polynucleotide encoded by said gene nonfunctional upon introduction into a host cell.
29. The nucleic acid of any one of items 1-28, wherein said nucleic acid further comprises a transgene.
30. The nucleic acid of item 29, wherein the transgene encodes a protein that provides improved oncolytic activity, a protein capable of eliciting an immune response for use as a vaccine, a therapeutic polypeptide or a therapeutic nucleic acid.
31. The nucleic acid of item 30, wherein the transgene encodes a protein that provides improved oncolytic activity.
32. The nucleic acid of item 30, wherein the transgene encodes a protein capable of eliciting an immune response for use as a vaccine.
33. The nucleic acid of item 30, wherein the transgene encodes a therapeutic polypeptide.
34. The nucleic acid of item 30, wherein the transgene encodes a therapeutic nucleic acid.
35. A recombinant vaccinia virus encoded by the vaccinia virus genome of any one of items 1-34.
36. The recombinant vaccinia virus of item 33, wherein said virus is a viral vector encoding at least one transgene.
37. The recombinant vaccinia virus vector of item 37, wherein said deletions are deletions of the entire gene encoding the corresponding protein.
38. The recombinant vaccinia virus vector of any one of items 37-38, wherein each of said deletions is a deletion of a portion of the gene, and wherein said deletion is sufficient to render said polynucleotide encoded by said gene nonfunctional upon introduction into a host cell.
39. The recombinant vaccinia virus vector of any one of items 38-40, wherein said vector further comprises a transgene encoding a protein that provides improved oncolytic activity, a protein capable of eliciting an immune response for use as a vaccine, a therapeutic polypeptide or a therapeutic nucleic acid or a protein that provides improved oncolytic immune activity.
40. The recombinant vaccinia virus vector of item 39, wherein the transgene encodes a protein that provides improved oncolytic activity.
41. The recombinant vaccinia virus vector of item 39, wherein the transgene encodes a protein capable of eliciting an immune response for use as a vaccine.
42. The recombinant vaccinia virus vector of item 39, wherein the transgene encodes a therapeutic polypeptide.
43. The recombinant vaccinia virus vector of item 39, wherein the transgene encodes a therapeutic nucleic acid.
44. The recombinant vaccinia virus vector of item 39, wherein the transgene encodes a protein that provides improved oncolytic immune activity.
45. The recombinant vaccina virus of any of items 37-44, wherein the vector is CopMD5p3p.
46. The nucleic acid of item 1, or the recombinant vaccinia virus vector of item 37, wherein upon contacting a population of mammalian cells with said nucleic acid or said recombinant vaccinia virus vector, the cells exhibit increased syncytia formation relative to a population of mammalian cells of the same type contacted with a form of the vaccinia virus vector that does not comprise said deletions.
47. The nucleic acid of item 1, or the recombinant vaccinia virus vector of item 37, wherein upon contacting a population of mammalian cells with said nucleic acid or said recombinant vaccinia virus vector, the cells exhibit increased spreading of the vaccinia virus vector relative to a population of mammalian cells of the same type contacted with a form of the vaccinia virus vector that does not comprise said deletions.
48. The nucleic acid of item 1, or the recombinant vaccinia virus of item 36, wherein said nucleic acid or said recombinant vaccinia virus vector exerts an increased cytotoxic effect on a population of mammalian cells relative to that of a form of the vaccinia virus vector that does not comprise said deletions.
49. The nucleic acid or the recombinant vaccinia virus vector of any one of items 46-48, wherein said mammalian cells are human cells.
50. The nucleic acid or the recombinant vaccinia virus vector of item 49, wherein said human cells are cancer cells.
51. A packaging cell line comprising the nucleic acid of any one of items 1-34, or the recombinant vaccinia virus vector of item 37.
52. A method of treating cancer in a mammalian patient, said method comprising administering a therapeutically effective amount of the nucleic acid of item 1, or the recombinant vaccinia virus of item 36 to said patient.
53. The method of item 52, wherein said mammalian patient is a human patient.
54. The method of item 52 or 53, wherein said cancer is selected from the group consisting of leukemia, lymphoma, liver cancer, bone cancer, lung cancer, brain cancer, bladder cancer, gastrointestinal cancer, breast cancer, cardiac cancer, cervical cancer, uterine cancer, head and neck cancer, gallbladder cancer, laryngeal cancer, lip and oral cavity cancer, ocular cancer, melanoma, pancreatic cancer, prostate cancer, colorectal cancer, testicular cancer, and throat cancer.
55. The method of item 52 or 53, wherein said cancer is selected from the group consisting of acute lymphoblastic leukemia (ALL), acute myeloid leukemia (AML), chronic lymphocytic leukemia (CLL), chronic myelogenous leukemia (CML), adrenocortical carcinoma, AIDS-related lymphoma, primary CNS lymphoma, anal cancer, appendix cancer, astrocytoma, atypical teratoid/rhabdoid tumor, basal cell carcinoma, bile duct cancer, extrahepatic cancer, ewing sarcoma family, osteosarcoma and malignant fibrous histiocytoma, central nervous system embryonal tumors, central nervous system germ cell tumors, craniopharyngioma, ependymoma, bronchial tumors, burkitt lymphoma, carcinoid tumor, primary lymphoma, chordoma, chronic myeloproliferative neoplasms, colon cancer, extrahepatic bile duct cancer, ductal carcinoma in situ (DCIS), endometrial cancer, ependymoma, esophageal cancer, esthesioneuroblastoma, extracranial germ cell tumor, extragonadal germ cell tumor, fallopian tube cancer, fibrous histiocytoma of bone, gastrointestinal carcinoid tumor, gastrointestinal stromal tumors (GIST), testicular germ cell tumor, gestational trophoblastic disease, glioma, childhood brain stem glioma, hairy cell leukemia, hepatocellular cancer, langerhans cell histiocytosis, hodgkin lymphoma, hypopharyngeal cancer, islet cell tumors, pancreatic neuroendocrine tumors, wilms tumor and other childhood kidney tumors, langerhans cell histiocytosis, small cell lung cancer, cutaneous T cell lymphoma, intraocular melanoma, merkel cell carcinoma, mesothelioma, metastatic squamous neck cancer, midline tract carcinoma, multiple endocrine neoplasia syndromes, multiple myeloma/plasma cell neoplasm, myelodysplastic syndromes, nasal cavity and paranasal sinus cancer, nasopharyngeal cancer, neuroblastoma, non-hodgkin lymphoma (NHL), non-small cell lung cancer (NSCLC), epithelial ovarian cancer, germ cell ovarian cancer, low malignant potential ovarian cancer, pancreatic neuroendocrine tumors, papillomatosis, paraganglioma, paranasal sinus and nasal cavity cancer, parathyroid cancer, penile cancer, pharyngeal cancer, pheochromocytoma, pituitary tumor, pleuropulmonary blastoma, primary peritoneal cancer, rectal cancer, retinoblastoma, rhabdomyosarcoma, salivary gland cancer, kaposi sarcoma, rhabdomyosarcoma, sezary syndrome, small intestine cancer, soft tissue sarcoma, throat cancer, thymoma and thymic carcinoma, thyroid cancer, transitional cell cancer of the renal pelvis and ureter, urethral cancer, endometrial uterine cancer, uterine sarcoma, vaginal cancer, vulvar cancer, and Waldenström macroglobulinemia.
56. A kit comprising the nucleic acid of item 1 or the recombinant vaccinia virus of items 36 and a package insert instructing a user of said kit to express said nucleic acid or said vector in a host cell.
57. A kit comprising the nucleic acid of item 1 or the recombinant vaccinia virus of items 36 and a package insert instructing a user to administer a therapeutically effective amount of said nucleic acid or recombinant vaccinia virus vector to a mammalian patient having cancer, thereby treating said cancer.
58. The kit of item 57, wherein said mammalian patient is a human patient.
59. The nucleic acid or recombinant vaccinia virus of any of the preceding items wherein the B8R gene is deleted.
60. The nucleic acid or recombinant vaccinia virus of item 59, wherein at least one transgene is inserted into the locus of the deleted B8R gene.
61. The nucleic acid or recombinant vaccinia virus of item 60, wherein at least two transgenes are inserted into the locus of the deleted B8R gene.
62. The nucleic acid or recombinant vaccinia virus of item 61, wherein at least three transgenes are inserted into the locus of the deleted B8R gene.
63. The nucleic acid or recombinant vaccinia virus of any one of items 59-62, wherein at least one additional transgene is inserted at locus that is not the locus of the B8R gene.
64. The nucleic acid or recombinant vaccinia virus of item 63, wherein the locus is the boundary of the 5p deletion.
65. The nucleic acid or recombinant vaccinia virus of item 64, wherein the locus is the boundary of the 3p deletion.

## Claims

1. A nucleic acid comprising a recombinant vaccinia virus genome, said recombinant vaccinia virus genome derived from the Vaccinia Copenhagen strain genome, wherein said recombinant vaccinia virus genome comprises a deletion in each of the following vaccinia genes: C2L, C1L, NIL, N2L, MIL, M2L, K1L, K2L, K3L, K4L, K5L, K6L, K7R, F1L, F2L, F3L, B14R, B15R, B16R, B17L, B18R, B19R, and B20R genes; and B21R, B22R, B23R, B24R, B25R, B26R, B27R, B28R, and B29R genes.

2. The nucleic acid of claim 1, wherein each of said deletions is a deletion of at least a portion of the polynucleotide encoding the corresponding gene, and wherein said deletion is sufficient to render said gene nonfunctional upon introduction into a host cell.

3. The nucleic acid of claim 1 or 2, wherein said nucleic acid further comprises a transgene.

4. The nucleic acid of claim 3, wherein the transgene encodes a protein that provides improved oncolytic activity, a protein capable of eliciting an immune response for use as a vaccine, a therapeutic polypeptide or a therapeutic nucleic acid.

5. The nucleic acid of any one of claims 1 to 4, wherein the recombinant vaccinia virus genome further comprises a deletion of the B8R gene.

6. The nucleic acid of claim 5, wherein at least one transgene, at least two transgenes, or at least three transgene(s) is/are inserted into the locus of the deleted B8R gene.

7. The nucleic acid of claim 5 or 6, wherein at least one additional transgene is inserted at a locus that is not the locus of the B8R gene.

8. The nucleic acid of claim 7, wherein the nucleic acid comprises a 5p deletion and the locus that is not the locus of the B8R gene is the boundary of the 5p deletion; or wherein the nucleic acid comprises a 3p deletion and the locus that is not the locus of the B8R gene is the boundary of the 3p deletion.

9. The nucleic acid of anyone of claims 1 to 8, wherein the TK gene of said recombinant vaccinia virus genome is not deleted

10. A recombinant vaccinia virus vector encoded by the vaccinia virus genome of claim 1 to 9.

11. The nucleic acid of any one of claims 1 to 9, or the recombinant vaccinia virus of claim 10, wherein:
(a) upon contacting a population of mammalian cells with said nucleic acid or said recombinant vaccinia virus, the population of mammalian cells exhibits:
increased syncytia formation relative to a population of mammalian cells of the same type contacted with a form of the nucleic acid or recombinant vaccinia virus that does not comprise said deletions; or
increased spreading of the recombinant vaccinia virus relative to a population of mammalian cells of the same type contacted with a form of the recombinant vaccinia virus that does not comprise said deletions;
or
(b) said nucleic acid or said recombinant vaccinia virus exerts an increased cytotoxic effect on a population of mammalian cells relative to that of a form of the recombinant vaccinia virus that does not comprise said deletions,
optionally wherein said population of mammalian cells are human cells; further optionally wherein said human cells are cancer cells.

12. A packaging cell line comprising the nucleic acid or recombinant vaccinia virus of any one of claims 1 to 11.

13. A nucleic acid or recombinant vaccinia virus as defined in any one of claims 1 to 11 for use in a method of treating cancer in a mammalian patient, said method comprising administering a therapeutically effective amount of said nucleic acid or said recombinant vaccinia virus to said mammalian patient.

14. The nucleic acid or recombinant vaccinia virus for use according to claim 13, wherein said mammalian patient is a human patient; and/or
wherein said cancer is selected from leukemia, lymphoma, liver cancer, bone cancer, lung cancer, brain cancer, bladder cancer, gastrointestinal cancer, breast cancer, cardiac cancer, cervical cancer, uterine cancer, head and neck cancer, gallbladder cancer, laryngeal cancer, lip and oral cavity cancer, ocular cancer, melanoma, pancreatic cancer, prostate cancer, colorectal cancer, testicular cancer, and throat cancer;
more particularly wherein said cancer is selected from acute lymphoblastic leukemia (ALL), acute myeloid leukemia (AML), chronic lymphocytic leukemia (CLL), chronic myelogenous leukemia (CML), adrenocortical carcinoma, AIDS-related lymphoma, primary CNS lymphoma, anal cancer, appendix cancer, astrocytoma, atypical teratoid/rhabdoid tumor, basal cell carcinoma, bile duct cancer, extrahepatic cancer, ewing sarcoma family, osteosarcoma and malignant fibrous histiocytoma, central nervous system embryonal tumors, central nervous system germ cell tumors, craniopharyngioma, ependymoma, bronchial tumors, burkitt lymphoma, carcinoid tumor, primary lymphoma, chordoma, chronic myeloproliferative neoplasms, colon cancer, extrahepatic bile duct cancer, ductal carcinoma in situ (DCIS), endometrial cancer, ependymoma, esophageal cancer, esthesioneuroblastoma, extracranial germ cell tumor, extragonadal germ cell tumor, fallopian tube cancer, fibrous histiocytoma of bone, gastrointestinal carcinoid tumor, gastrointestinal stromal tumors (GIST), testicular germ cell tumor, gestational trophoblastic disease, glioma, childhood brain stem glioma, hairy cell leukemia, hepatocellular cancer, langerhans cell histiocytosis, hodgkin lymphoma, hypopharyngeal cancer, islet cell tumors, pancreatic neuroendocrine tumors, wilms tumor and other childhood kidney tumors, langerhans cell histiocytosis, small cell lung cancer, cutaneous T cell lymphoma, intraocular melanoma, merkel cell carcinoma, mesothelioma, metastatic squamous neck cancer, midline tract carcinoma, multiple endocrine neoplasia syndromes, multiple myeloma/plasma cell neoplasm, myelodysplastic syndromes, nasal cavity and paranasal sinus cancer, nasopharyngeal cancer, neuroblastoma, non-hodgkin lymphoma (NHL), non-small cell lung cancer (NSCLC), epithelial ovarian cancer, germ cell ovarian cancer, low malignant potential ovarian cancer, pancreatic neuroendocrine tumors, papillomatosis, paraganglioma, paranasal sinus and nasal cavity cancer, parathyroid cancer, penile cancer, pharyngeal cancer, pheochromocytoma, pituitary tumor, pleuropulmonary blastoma, primary peritoneal cancer, rectal cancer, retinoblastoma, rhabdomyosarcoma, salivary gland cancer, kaposi sarcoma, rhabdomyosarcoma, sezary syndrome, small intestine cancer, soft tissue sarcoma, throat cancer, thymoma and thymic carcinoma, thyroid cancer, transitional cell cancer of the renal pelvis and ureter, urethral cancer, endometrial uterine cancer, uterine sarcoma, vaginal cancer, vulvar cancer, and Waldenstrom macroglobulinemia.

15. A kit comprising the nucleic acid or recombinant vaccinia virus of any one of claims 1 to 11 and a package insert instructing a user of said kit to: (a) express said nucleic acid or said recombinant vaccinia virus in a host cell; or (b) administer a therapeutically effective amount of said nucleic acid or recombinant vaccinia virus to a mammalian patient having cancer, thereby treating said cancer; optionally wherein said mammalian patient is a human patient.
